(19) Europäisches Patentamt
European Patent Office
Office européen des brevets

(11) EP 4 691 487 A1

(12) EUROPEAN PATENT APPLICATION
published in accordance with Art. 153(4) EPC

(43) Date of publication:
**11.02.2026 Bulletin 2026/07**

(21) Application number: **24778252.7**

(22) Date of filing: **29.03.2024**

(51) International Patent Classification (IPC):
***A61K 39/395** (2006.01)* ***A61P 35/00** (2006.01)*

(52) Cooperative Patent Classification (CPC):
**A61K 39/395; A61P 35/00**

(86) International application number:
**PCT/CN2024/084887**

(87) International publication number:
**WO 2024/199466 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 CN 202310343152**
**28.09.2023 CN 202311276741**
**12.03.2024 CN 202410283445**

(71) Applicant: **Inxmed (Nanjing) Co., Ltd.**
**Nanjing, Jiangsu 210061 (CN)**

(72) Inventors:
- **ZHANG, Baoyuan**
**Nanjing, Jiangsu 210061 (CN)**
- **LIU, Xuebin**
**Nanjing, Jiangsu 210061 (CN)**
- **PANG, Ran**
**Nanjing, Jiangsu 210061 (CN)**
- **WANG, Zaiqi**
**Nanjing, Jiangsu 210061 (CN)**

(74) Representative: **D Young & Co LLP**
**3 Noble Street**
**London EC2V 7BQ (GB)**

# (54) METHOD FOR INCREASING CONCENTRATION OF MACROMOLECULAR DRUG IN TUMOR TISSUE

(57) Provided are combination therapy of tumors using IN10018 and a macromolecular drug. The macromolecular drug is a monoclonal antibody, a bispecific antibody, or an antibody conjugate.



Processed by Luminess, 75001 PARIS (FR)

## Description

The present application claims the priority of Chinese application No. 202310343152.0 filed on March 31, 2023, Chinese application No. 202311276741.8 filed on September 28, 2023, and Chinese application No. 202410283445.9 filed on March 12, 2024.

## TECHNICAL FIELD

The present invention belongs to the field of medicinal chemistry. Specifically, the present invention relates to the combined use of the Focal Adhesion Kinase (FAK) inhibitor IN10018 and a macromolecular drug for the treatment of tumors.

## BACKGROUND

Malignant tumors (cancer) have become one of the major public health issues posing a serious threat to global health. According to the latest statistics, malignant tumor deaths account for 23.91% of all causes of death in China, and the incidence and mortality of malignant tumors have been steadily increasing over the past decade. Annual medical expenses related to malignant tumors in China exceed 220 billion CNY.
In recent years, the research and development process of macromolecular drugs, primarily monoclonal antibodies, bispecific antibodies, and antibody conjugates, has been gradually accelerated, with numerous macromolecular drugs entering the anti-cancer treatment market. Macromolecular drugs can precisely identify tumor antigens and precisely kill tumor cells through blocking the cascade reaction between the antigen and its downstream or through the small-molecule conjugates carried, with such unique mechanism to expand effective anti-tumor activity. Tumor tissue includes tumor cells and the surrounding stroma, and there are numerous mechanisms that hinder the diffusion (penetration) of macromolecular drugs in these stroma-rich tumor microenvironments. These stroma barriers, including fibroblasts and fibrotic collagen, which are in close contact with tumor cells, further hinder the effective effect of anti-cancer drugs to tumor cells. Based on this mechanism, an agent capable of inhibiting the proliferation of tumor fibrosis or reducing the fibrotic stroma may help increase the concentration of macromolecular drugs in tumor tissue. Therefore, the development of a drug capable of inhibiting the proliferation of tumor fibrosis or reducing the fibrotic stroma may promote the treatment of tumor and will have important clinical value.

## SUMMARY

The inventors found that the focal adhesion kinase (FAK) inhibitor IN10018 contribute to increase the concentration of macromolecular drugs in tumor tissue. Without wishing to be bound by theory, it is believed that activated FAK pathways can significantly promote tumor fibrosis, and that the FAK inhibitor IN10018 can reduce FAK activity and significantly reduce tumor fibrosis hyperplasia, thereby contributing to the penetration of macromolecular drugs into the tumor microenvironment.
In one aspect, the present disclosure provides the use of IN10018 or a pharmaceutically acceptable salt thereof in the manufacture of a medicament for increasing the concentration of a macromolecular drug in tumor tissue, wherein the macromolecular drug is a monoclonal antibody, a bispecific antibody, or an antibody conjugate, the structure of IN10018 is as follows:

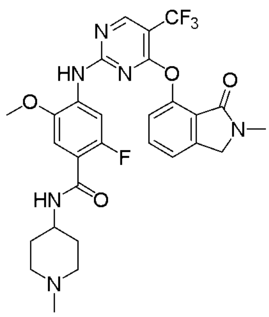

.

In another aspect, the present disclosure provides a pharmaceutical combination product comprising IN10018 or a pharmaceutically acceptable salt thereof and a macromolecular drug, for use in treating a tumor in a subject, wherein the macromolecular drug is a monoclonal antibody, a bispecific antibody, or an antibody conjugate, the structure of IN10018 is as follows:

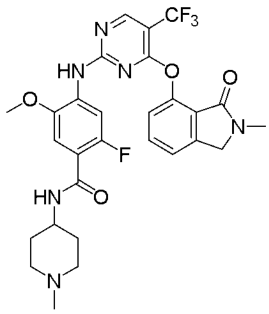

.

In another aspect, the present disclosure provides the use of IN10018 or a pharmaceutically acceptable salt thereof for increasing the concentration of a macromolecular drug in tumor tissue, wherein the macromolecular drug is a monoclonal antibody, a bispecific antibody, or an antibody conjugate, the structure of IN10018 is as follows:

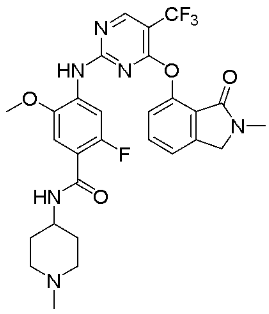

.

In another aspect, the present disclosure provides a method for treating tumors, comprising administering a therapeutically effective amount of IN10018 or a pharmaceutically acceptable salt thereof and a macromolecular drug to a subject in need thereof, wherein the macromolecular drug is a monoclonal antibody, a bispecific antibody, or an antibody conjugate, the structure of IN10018 is as follows:

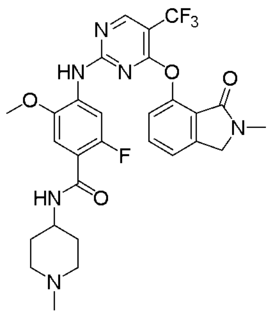

.

In another aspect, the present disclosure provides a kit or a pharmaceutically acceptable composition comprising:

IN10018 or a pharmaceutically acceptable salt thereof; and
a macromolecular drug, wherein the macromolecular drug is a monoclonal antibody, a bispecific antibody, or an antibody conjugate;
the structure of IN10018 is as follows:

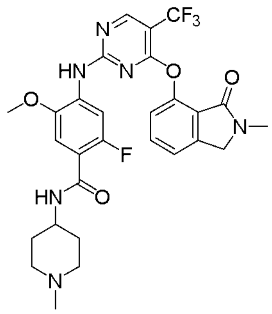

.

In another aspect, the present disclosure provides a pharmaceutical combination product comprising IN10018 or a pharmaceutically acceptable salt thereof and a macromolecular drug, for treating a tumor in a subject, wherein the macromolecular drug is a monoclonal antibody, a bispecific antibody or an antibody conjugate.

In another aspect, the present disclosure provides the use of IN10018 or a pharmaceutically acceptable salt thereof and a macromolecular drug in the manufacture of a combination medicament for treating tumors, wherein the macromolecular drug is a monoclonal antibody, a bispecific antibody, or an antibody conjugate.

In another aspect, the present disclosure provides the use of IN10018 or a pharmaceutically acceptable salt thereof in the manufacture of a medicament for treating tumors in combination with a macromolecular drug, wherein the macromolecular drug is a monoclonal antibody, a bispecific antibody, or an antibody conjugate.

In another aspect, the present disclosure provides the use of a macromolecular drug in the manufacture of a medicament for treating tumors in combination with IN10018 or a pharmaceutically acceptable salt thereof, wherein the macromolecular drug is a monoclonal antibody, a bispecific antibody, or an antibody conjugate.

In another aspect, the present disclosure provides a kit comprising: IN10018 or a pharmaceutically acceptable salt thereof; and an instruction indicating that IN10018 or a pharmaceutically acceptable salt thereof can be used for treating tumors in combination with a macromolecular drug, wherein the macromolecular drug is a monoclonal antibody, a bispecific antibody, or an antibody conjugate.

In another aspect, the present disclosure provides a kit comprising: a macromolecular drug; and an instruction indicating that the macromolecular drug can be used for treating tumors in combination with IN10018 or a pharmaceutically acceptable salt thereof; the macromolecular drug is a monoclonal antibody, a bispecific antibody, or an antibody conjugate.

Optionally, the pharmaceutically acceptable salt of IN10018 is tartrate.

Optionally, the monoclonal antibody is Racotumomab, Rituximab, 131I-Metuximab, JMT-103, Necitumumab, Alemtuzumab, Elotuzumab, Bevacizumab, Ofatumumab, Tocilizumab, Atezolizumab, Toripalimab, HX-008, Camrelizumab, Ocrelizumab, Sugemalimab, Lenzilumab, Sintilimab, Vilobelimab, Margetuximab, Siltuximab, Mogamulizumab, amivantamab, Cadonilimab, Inebilizumab, iodine I 131 derlotuximab biotin, Isatuximab, Serplulimab, Retifanlimab, Cetuximab, Adebrelimab, Tislelizumab, Penpulimab, Teprotumumab, Itolizumab, Dostarlimab, Denosumab, Obinutuzumab, Nimotuzumab, Teclistamab, Daratumumab, dinutuximab, Tafasitamab, Socazolimab, Dupilumab, Prolgolimab, Blinatumomab, Geptanolimab, Panitumumab, Canakinumab, Ramucirumab, envafolimab, Belimumab, Leronlimab, Ranibizumab, Natalizumab, Cosibelimab, Zimberelimab, Trastuzumab, Catumaxomab, Durvalumab, avelumab, Ublituximab, Cemiplimab, Pembrolizumab, Glofitamab, Pertuzumab, Bermekimab, tremelimumab, ipilimumab, Naxitamab, infliximab, nivolumab, Omburtamab, Crizanlizumab, burosumab, Talquetamab, Besilesomab, Alirocumab, Arcitumomab, or a biosimilar thereof; particularly, Trastuzumab or a biosimilar thereof; preferably, Trastuzumab.

Biosimilars, also known as biosimilar products, are biological drugs that are highly similar to approved original biological drugs in terms of quality, safety, and effectiveness. For example, Bevacizumab biosimilar, Adalimumab biosimilar, and Rituximab biosimilar.

Optionally, the bispecific antibody is Teclistamab, Blinatumomab, Cadonilimab, Mosunetuzumab, Catumaxomab, Ublituximab, amivantamab, Talquetamab, Epcoritamab, Glofitamab, Zanidatamab, Elranatamab, Tebotelimab, amivantamab, SI-B001, Odronextamab, KN-026, KN-046, Ivonescimab, SHR-1701, M7824, GEN-3009, Navicixizumab, GB-261, CM-355, Plamotamab, or a biosimilar thereof.

Optionally, the antibody conjugate is Loncastuximab Tesirine, Ibritumomab Tiuxetan, Tisotumab Vedotin, Sacituzumab Govitecan, Enfortumab Vedotin, Inotuzumab Ozogamicin, Gemtuzumab Ozogamicin, Belantamab Mafodotin, Trastuzumab Emtansine, Moxetumomab Pasudotox, Polatuzumab Vedotin, Disitamab Vedotin, Brentuximab Vedotin, Trastuzumab deruxtecan(DS-8201), Trastuzumab Emtansine, Cetuximab Sarotalocan, Mirvetuximab Soravtansine, Trastuzumab Duocarmazine, ARX-788, KL-A264, Upifitamab Rilsodotin, Tusamitamab Ravtansine, Naptumomab Estafenatox, Datopotamab Deruxtecan, Oportuzumab Monatox, SHR-A1811, Patritumab Deruxtecan, Telisotuzumab vedotin, Trastuzumab Duocarmazine, MK-2140, TAA-013, BIO-106, DB-1305, MRG-004A, AZD-8205, ADCT-602, FOR-46, TPX-4589, BMS-986148, SOT-102, ESG-401, CD117-ADC, LCB-14, W-0101, REGN-5093-M114, CX-2029, BDC-1001, DGN549-C (Pivekimab Sunirine), AVID-100, MRG-003, CAT-5001, HDP-101, MORAb-202 (Farletuzumab

Ecteribulin), L-DOS47, Praluzatamab Ravtansine, BA3021(Ozuriftamab Vedotin), DS-7300, BA3011(Mecbotamab Vedotin), Camidanlumab Tesirine, Ladiratuzumab Vedotin, LMB-2, SAT-012, DEBIO-1562, PSMA-ADC, MRG-002, OBI-999, Coltuximab Ravtansine, KL-A166, DX-126-262, NBE-002, OXS-1550, Lorvotuzumab Mertansine, TAC-001, MT-8633, TRPH-222, TAK-164, STRO-001, CX-2043, TBL-0306M, HDP-103, DAN-311, PRO-1102, GM-103 ADC, TE-1218, TE-1112, T-PNU, BV-001, DS-1062, SKB264, Ab6000-Dxd, or a biosimilar thereof; particularly, ESG-401, DS-1062, SKB264, Sacituzumab Govitecan, Trastuzumab deruxtecan(DS-8201), Ab6000-Dxd, or a biosimilar thereof; preferably, ESG-401, DS-1062, SKB264, Sacituzumab Govitecan / Trastuzumab deruxtecan(DS-8201), or Ab6000-Dxd; preferably, ESG-401, Sacituzumab Govitecan, Trastuzumab deruxtecan(DS-8201) or Ab6000-Dxd.

Optionally, the macromolecular drug is an antibody conjugate.

Optionally, the antibody conjugate is Loncastuximab Tesirine, Ibritumomab Tiuxetan, Tisotumab Vedotin, Sacituzumab Govitecan, Enfortumab Vedotin, Inotuzumab Ozogamicin, Gemtuzumab Ozogamicin, Belantamab Mafodotin, Trastuzumab Emtansine, Moxetumomab Pasudotox, Polatuzumab Vedotin, Disitamab Vedotin, Brentuximab Vedotin, Trastuzumab deruxtecan(DS-8201), Trastuzumab Emtansine, Cetuximab Sarotalocan, Mirvetuximab Soravtansine, Trastuzumab Duocarmazine, ARX-788, KL-A264, Upifitamab Rilsodotin, Tusamitamab Ravtansine, Naptumomab Estafenatox, Datopotamab Deruxtecan, Oportuzumab Monatox, SHR-A1811, Patritumab Deruxtecan, Telisotuzumab vedotin, Trastuzumab Duocarmazine, MK-2140, TAA-013, BIO-106, DB-1305, MRG-004A, AZD-8205, ADCT-602, FOR-46, TPX-4589, BMS-986148, SOT-102, ESG-401, CD117-ADC, LCB-14, W-0101, REGN-5093-M114, CX-2029, BDC-1001, DGN549-C (Pivekimab Sunirine), AVID-100, MRG-003, CAT-5001, HDP-101, MORAb-202 (Farletuzumab Ecteribulin), L-DOS47, Praluzatamab Ravtansine, BA3021(Ozuriftamab Vedotin, DS-7300, BA3011(Mecbotamab Vedotin), Camidanlumab Tesirine, Ladiratuzumab Vedotin, LMB-2, SAT-012, DEBIO-1562, PSMA-ADC, MRG-002, OBI-999, Coltuximab Ravtansine, KL-A166, DX-126-262, NBE-002, OXS-1550, Lorvotuzumab Mertansine, TAC-001, MT-8633, TRPH-222, TAK-164, STRO-001, CX-2043, TBL-0306M, HDP-103, DAN-311, PRO-1102, GM-103 ADC, TE-1218, TE-1112, T-PNU, BV-001, DS-1062, SKB264, or a biosimilar thereof; particularly, ESG-401, DS-1062, SKB264, Sacituzumab Govitecan, Ab6000-Dxd, or a biosimilar thereof; preferably, ESG-401, DS-1062, SKB264, Sacituzumab Govitecan, Trastuzumab deruxtecan(DS-8201) or Ab6000-Dxd; preferably, ESG-401, Sacituzumab Govitecan, Trastuzumab deruxtecan(DS-8201) or Ab6000-Dxd.

Optionally, the antibody conjugate is a Trop-2 antibody conjugate.

Optionally, the Trop-2 antibody conjugate is ESG-401, DS-1062, SKB264, Sacituzumab Govitecan, or a biosimilar thereof; preferably, ESG-401, DS-1062, SKB264 or Sacituzumab Govitecan; preferably, ESG-401 or Sacituzumab Govitecan.

Optionally, the IN10018 or a pharmaceutically acceptable salt thereof and the macromolecular drug are administered to the subject simultaneously or sequentially.

Optionally, the above-mentioned use, pharmaceutical combination product, method, kit or pharmaceutically acceptable composition is used for treating tumors.

Optionally, the tumor is selected from bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, bile duct cancer, leiomyosarcoma, liposarcoma, nasopharyngeal cancer, neuroendocrine cancer, ovarian cancer, salivary gland cancer, metastatic tumor caused by spindle cell carcinoma, anaplastic large cell lymphoma, undifferentiated thyroid cancer, non-Hodgkin's lymphoma, Hodgkin's lymphoma, glioma and hematological malignancies, such as acute myeloid leukemia (AML), acute lymphocytic leukemia (ALL), diffuse large B-cell lymphoma (DLBCL), follicular lymphoma (FL), chronic lymphocytic leukemia (CLL), chronic granulocytic leukemia (CML); preferably, the tumor is gastric cancer, lung cancer, breast cancer, glioma, esophageal cancer, pancreatic cancer, head and neck cancer, colon cancer or ovarian cancer; more preferably, the tumor is gastric cancer, breast cancer, pancreatic cancer, colon cancer, ovarian cancer or lung cancer.

## BRIEF DESCRIPTION OF DRAWINGS

To illustrate the technical solutions of the examples in the present disclosure more clearly, the drawings of the examples will be briefly introduced below. Obviously, the drawings in the following description only relate to some examples of the present disclosure, and are not intended to limit the present invention.

FIG. 1 shows whole-slide scan thumbnails of a frozen section in Example 1. Images (thumbnail) were obtained by scanning the entire section using a digital pathology imaging whole-slide scanning system, blue represents DAPI-stained cell nuclei, and green represents GFP protein fluorescence from the administered drug T-GFP itself. A and B are whole-slide scan figures of two animals in the control group, respectively; C and D are whole-slide scan figures of

two animals in the IN10018+T-GFP group, respectively.

FIG. 2 shows the percentage of T-GFP-positive cells in Example 1. The percentage of tumor infiltration level of the test article T-GFP in a subcutaneous xenograft tumor model of human gastric cancer NCI-N87 cells in Balb/c-nude mice after administration of IN10018 was shown.

FIG. 3 shows the H-Score of T-GFP-positive cells in Example 1. The H-Score of the tumor infiltration level of the test article T-GFP in a subcutaneous xenograft tumor model of human gastric cancer NCI-N87 cells in Balb/c-nude mice after administration of IN10018 was shown.

FIG. 4 shows the changes in body weight in different groups of MDA-MB-468 tumor-bearing mice after administration in Example 2. The data are expressed as mean + standard error (SEM).

FIG. 5 shows the relative body weight changes in different groups of MDA-MB-468 tumor-bearing mice after administration in Example 2. The data are expressed as mean + standard error (SEM).

FIG. 6 shows the changes in tumor volume in MDA-MB-468 tumor-bearing mice during the administration period in Example 2. The data are expressed as mean + standard error (SEM).

FIG. 7 shows whole-slide scan thumbnails of the frozen section in Example 3.

FIG. 8 shows the tumor growth curves of mice after administration of different test articles in Example 4. The data points represent the mean tumor volume within the group, and the error bars represent the standard error (SEM).

FIG. 9 shows the binding of tumor cells to antibodies in each treatment group at the end point of the experiment in Example 5. The staining intensity represents the binding intensity of tumor cells to antibodies in each group.

FIG. 10 shows the data on the day of grouping and administration, 14 days after inoculation in Example 6.

FIG. 11 shows the total fluorescence intensity in Example 6. The data points represent the total fluorescence intensity of each animal in the group, and the error bars represent the standard error (SEM).

FIG. 12 shows the fluorescence imaging of H-Cy5.5 injection in Example 6, wherein A and B represent the fluorescence images of the G1 control group, C and D represent the fluorescence images of the G2 IN10018 25mg/kg group, and E represents the fluorescence image of the negative control group.

FIG. 13 shows the average fluorescence intensity curve of the tumor site of the test article H-Cy5.5 in a subcutaneous xenograft model of human gastric cancer NCI-N87 cells in Balb/c-nude mice after the administration of IN10018 in Example 6. The value at 0h represents the average fluorescence intensity of the negative control group. Data points represent the average fluorescence intensity in the group, and error bars represent the standard error (SEM).

FIG. 14 shows the fluorescence imaging of the test article H-Cy5.5 in a subcutaneous xenograft tumor model of human gastric cancer NCI-N87 cells in Balb/c-nude mice after injection for 48h in Example 6. A and B represent fluorescence images of the G1 control group, while C and D represent fluorescence images of the G2 IN10018 25 mg/kg group.

FIG. 15 shows the tumor growth curves following treatment in the BALB/c-nude mouse subcutaneous xenograft model co-inoculated with NCI-N87&NIH-3T3 human gastric cancer cells and mouse embryonic fibroblasts in Example 7. The data points represent the mean tumor volume in the group, and the error bars represent the standard error (SEM).

FIG. 16 shows the body weight change curves following treatment in the BALB/c-nude mouse subcutaneous xenograft model co-inoculated with NCI-N87&NIH-3T3 human gastric cancer cells and mouse embryonic fibroblasts in Example 7. The data points represent the mean body weight in the group, and the error bars represent the standard error (SEM).

FIG. 17 shows the body weight change rate curves following treatment in the BALB/c-nude mouse subcutaneous xenograft model co-inoculated with NCI-N87&NIH-3T3 human gastric cancer cells and mouse embryonic fibroblasts in Example 7. The data points represent the mean body weight change rate in the group, and the error bars represent the standard error (SEM).

FIG. 18 shows the percent (%) change of body weight in Example 8. Data points represent the percent mean change between the groups in body weight, and error bars represent the standard error of the mean (SEM).

FIG. 19 shows the tumor growth curves in Example 8. Data points represent means between the groups, and error bars represent standard error of the mean (SEM).

FIG. 20 shows the tumor growth curve in Example 9.

FIG. 21 shows the tumor growth curve in Example 10.

FIG. 22 shows the tumor growth curve in Example 11.

FIG. 23 shows the tumor growth curve in Example 12.

## DETAILED DESCRIPTION

To make objects, technical details and advantages of the embodiments of the disclosure clearer, the technical solutions of the embodiments will be described below in a clearly and fully understandable way in conjunction with the drawings of the examples of the present disclosure. Apparently, the described embodiments are just a part but not all of the embodiments of the disclosure. Based on the described embodiments herein, those skilled in the art can obtain other

embodiment(s), without any inventive work, which should be within the scope of the disclosure.

The present disclosure can be implemented in other specific forms without departing from basic attributes of the present disclosure. It should be understood that any and all embodiments of the present disclosure can be combined with the technical features of any one or more other embodiments to obtain additional embodiments without conflict. The present disclosure comprises the additional embodiments obtained by such combination.

All the publications and patents mentioned in the present disclosure are hereby incorporated by reference in their entirety. If the use or terminology used in any publication and patent incorporated by reference conflicts with the use or terminology used in the present disclosure, the use and terminology of this disclosure shall prevail.

The section headings used herein are used for organizational purposes only and are not to be construed as limiting the subject matter described.

Unless otherwise specified, all technical and scientific terms used herein have the same meaning as those commonly used in the art to which the claimed subject matter belongs. If there are multiple definitions for a term, the definition herein shall prevail.

Unless in the working examples or otherwise indicated, all numbers stating quantitative properties such as dosage in the specification and claims should be understood as modified by the term "about" in all instances. Also, it should be understood that any numerical range listed in the present application are intended to encompass all the subranges within the range and any combination of various endpoints of the range or a subrange thereof.

The "comprise/comprising," "include/including," or "contain/containing," or similar terms are intended to specify that the elements stated before these terms encompass the elements and equivalents thereof listed after these terms, but do not preclude the elements not recited. The term "comprise," "include (contain)" used herein can be in open, semi-close, and close forms. In other words, the term also comprises "consisting essentially of" or "consisting of".

Definition

The following terms and symbols used in the present application have the meanings described below, unless the context indicates otherwise.

The "monoclonal antibody" used herein refers to monoclonal antibody drugs, which are generally biological preparations and are large molecular proteins, and their target is a specific protein on the cell membrane. In some embodiments, the monoclonal antibody is Racotumomab, Rituximab, 1311-Metuximab, JMT-103, Necitumumab, Alemtuzumab, Elotuzumab, Bevacizumab, Ofatumumab, Tocilizumab, Atezolizumab, Toripalimab, HX-008, Camrelizumab, Ocrelizumab, Sugemalimab, Lenzilumab, Sintilimab, Vilobelimab, Margetuximab, Siltuximab, Mogamulizumab, amivantamab, Cadonilimab, Inebilizumab, iodine I 131 derlotuximab biotin, Isatuximab, Serplulimab, Retifanlimab, Cetuximab, Adebrelimab, Tislelizumab, Penpulimab, Teprotumumab, Itolizumab, Dostarlimab, Denosumab, Obinutuzumab, Nimotuzumab, Teclistamab, Daratumumab, dinutuximab, Tafasitamab, Socazolimab, Dupilumab, Prolgolimab, Blinatumomab, Geptanolimab, Panitumumab, Canakinumab, Ramucirumab, envafolimab, Belimumab, Leronlimab, Ranibizumab, Natalizumab, Cosibelimab, Zimberelimab, Trastuzumab, Catumaxomab, Durvalumab, avelumab, Ublituximab, Cemiplimab, Pembrolizumab, Glofitamab, Pertuzumab, Bermekimab, tremelimumab, ipilimumab, Naxitamab, infliximab, nivolumab, Omburtamab, Crizanlizumab, burosumab, Talquetamab, Besilesomab, Alirocumab, Arcitumomab, or a biosimilar thereof; in some embodiments, the monoclonal antibody is Trastuzumab or a biosimilar thereof; in some embodiments, the monoclonal antibody is Trastuzumab.

As used herein, "Bispecific Antibody" (bsAb) refers to an antibody that have two specific antigen-binding sites. In some embodiments, the bispecific antibody is Teclistamab, Blinatumomab, Cadonilimab, Mosunetuzumab, Catumaxomab, Ublituximab, amivantamab, Talquetamab, Epcoritamab, Glofitamab, Zanidatamab, Elranatamab, Tebotelimab, amivantamab, SI-B001, Odronextamab, KN-026, KN-046, Ivonescimab, SHR-1701, M7824, GEN-3009, Navicixizumab, GB-261, CM-355, Plamotamab, or a biosimilar thereof.

As used herein, an "antibody-drug conjugate" (ADC) refers to a drug in which a biologically active small molecule drug is linked to a monoclonal antibody via a chemical linker, and the monoclonal antibody acts as a carrier to deliver the small molecule drug to target cells. In some embodiments, the macromolecular drug is an antibody conjugate. For example, the antibody conjugate is Loncastuximab Tesirine, Ibritumomab Tiuxetan, Tisotumab Vedotin, Sacituzumab Govitecan, Enfortumab Vedotin, Inotuzumab Ozogamicin, Gemtuzumab Ozogamicin, Belantamab Mafodotin, Trastuzumab Emtansine, Moxetumomab Pasudotox, Polatuzumab Vedotin, Disitamab Vedotin, Brentuximab Vedotin, Trastuzumab deruxtecan(DS-8201), Trastuzumab Emtansine, Cetuximab Sarotalocan, Mirvetuximab Soravtansine, Trastuzumab Duocarmazine, ARX-788, KL-A264, Upifitamab Rilsodotin, Tusamitamab Ravtansine, Naptumomab Estafenatox, Datopotamab Deruxtecan, Oportuzumab Monatox, SHR-A1811, Patritumab Deruxtecan, Telisotuzumab vedotin, Trastuzumab Duocarmazine, MK-2140, TAA-013, BIO-106, DB-1305, MRG-004A, AZD-8205, ADCT-602, FOR-46, TPX-4589, BMS-986148, SOT-102, ESG-401, CD117-ADC, LCB-14, W-0101, REGN-5093-M114, CX-2029, BDC-1001, DGN549-C (Pivekimab Sunirine), AVID-100, MRG-003, CAT-5001, HDP-101, MORAb-202 (Farletuzumab Ecteribulin), L-DOS47, Praluzatamab Ravtansine, BA3021(Ozuriftamab Vedotin), DS-7300, BA3011(Mecbotamab

Vedotin), Camidanlumab Tesirine, Ladiratuzumab Vedotin, LMB-2, SAT-012, DEBIO-1562, PSMA-ADC, MRG-002, OBI-999, Coltuximab Ravtansine, KL-A166, DX-126-262, NBE-002, OXS-1550, Lorvotuzumab Mertansine, TAC-001, MT-8633, TRPH-222, TAK-164, STRO-001, CX-2043, TBL-0306M, HDP-103, DAN-311, PRO-1102, GM-103 ADC, TE-1218, TE-1112, T-PNU, BV-001, Ab6000-Dxd, or a biosimilar thereof; in some embodiments, the antibody conjugate is ESG-401, Sacituzumab Govitecan, Trastuzumab deruxtecan (DS-8201), Ab6000-Dxd, or a biosimilar thereof; in some embodiments, the antibody conjugate is ESG-401, Sacituzumab Govitecan, or Trastuzumab deruxtecan (DS-8201), or Ab6000-Dxd.

Trastuzumab is an anti-Her 2 monoclonal antibody.

ESG-401 is an antibody conjugate targeting Trop-2, jointly developed by Shanghai Shijian Biotechnology Co., Ltd. and Suzhou Lianning Biopharmaceutical Co., Ltd., disclosed in WO2021225892A1.

Sacituzumab Govitecan is an antibody conjugate targeting Trop-2, developed by Immunomedics, with the trade name Trodelvy.

As used herein, "pharmaceutical combination" or "pharmaceutical combination product" can refer to a fixed combination in the form of one dosage unit (for example, all active pharmaceutical ingredients are present in one dosage form) or a product as a kit-of-parts for combined administration, or it may refer to a combination of one drug and instructions indicating that the drug can be used in combination with one or more other drugs.

As used herein, "combination therapy" or "combination drug" refers to the use of a drug in combination with one or more other drugs to treat a disease, including the combination of a drug with one or more other drugs and the combination of a drug with instructions indicating that the drug can be used in combination with one or more other drugs.

"Simultaneous or sequential administration" in the present application refers to the simultaneous or sequential administration at certain time intervals of more than two drugs within one administration cycle (e.g., within 4 weeks, within 3 weeks, within 2 weeks, within 1 week, or within 24 hours). The modes of drug administration (e.g., oral, intravenous, intramuscular, or subcutaneous administration, etc.) may be the same or different, and the dosing frequency/cycle of more than two drugs may be the same or different. When the treatment method, product, or use of the present disclosure involves two drugs, the two drugs may be administered simultaneously or separately at certain time intervals.

As used herein, the term "treat", "treating", or "treats" refers to administering one or more pharmaceutical substances to a subject suffering from a disease or having symptoms of the disease to cure, alleviate, relieve, alter, heal, ameliorate, improve, or affect the disease or symptoms of the disease. In some embodiments, the disease is a tumor or cancer.

As used herein, the term "tumor" refers to an abnormal lesion formed by the loss of normal regulation of local tissue cells' growth at the genetic level under the influence of various tumorigenic factors, resulting in clonal abnormal proliferation. The tumors include, but are not limited to: bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, bile duct cancer, leiomyosarcoma, liposarcoma, nasopharyngeal cancer, neuroendocrine cancer, ovarian cancer, salivary gland cancer, metastatic tumor caused by spindle cell carcinoma, anaplastic large cell lymphoma, undifferentiated thyroid cancer, non-Hodgkin's lymphoma, Hodgkin's lymphoma, glioma and hematological malignancies, such as acute myeloid leukemia (AML), acute lymphocytic leukemia (ALL), diffuse large B-cell lymphoma (DLBCL), follicular lymphoma (FL), chronic lymphocytic leukemia (CLL), chronic granulocytic leukemia (CML); preferably, the tumor is lung cancer, breast cancer, glioma, esophageal cancer, pancreatic cancer, head and neck cancer, colon cancer or ovarian cancer; more preferably, the tumor is gastric cancer, breast cancer, pancreatic cancer, colon cancer, ovarian cancer or lung cancer.

As used herein, the term "subject" refers to both mammals and non-mammals. Mammals refer to any member of the mammal family, including but not limited to human; non-human primate such as chimpanzee and other apes and monkey species; farm animals such as cattle, horse, sheep, goat, and pig; livestock such as rabbit, dog, and cat; laboratory animal, including rodent such as rat, mice, and guinea pig; and the like. Examples of non-mammals include, but are not limited to, birds. The term "subject" does not limit the subject to a particular age or sex. In some embodiments, the subject is a human.

As used herein, the term "pharmaceutically acceptable" means non-toxic, biologically tolerable, and suitable for administration to a subject.

As used herein, the term "pharmaceutically acceptable salt" refers to non-toxic, biologically tolerable acid addition salts suitable for administration to a subject, including but not limited to: acid addition salts formed with inorganic acids, such as hydrochlorides, hydrobromides, carbonates, bicarbonates, phosphates, sulfates, sulfites, nitrates, etc.; and acid addition salts formed with organic acids, such as formate, acetate, malate, maleate, fumarate, tartrate, succinate, citrate, lactate, methanesulfonate, p-toluenesulfonate, 2-hydroxyethanesulfonate, benzoate, salicylate, stearate, and salts formed with alkanedicarboxylic acids of the formula $HOOC-(CH_2)_n-COOH$ (wherein n is 0-4), etc.

In addition, pharmaceutically acceptable acid addition salts can be prepared by dissolving the free base in a suitable solvent and treating the solution with an acid according to conventional procedures for preparing acid addition salts from basic compounds. Those skilled in the art can readily identify various synthetic methods for preparing non-toxic pharmaceutically acceptable acid addition salts without undue experimentation. In some embodiments, the pharmaceutically acceptable salt of IN10018 is a tartrate salt.

As used herein, the term "pharmaceutically acceptable composition" means that it must be chemically and/or toxicologically compatible with the other ingredients comprising the Preparation, and/or compatible with the subject being treated therewith. The term "therapeutically effective amount" as used herein means an amount that is generally sufficient to produce a beneficial therapeutic effect on the subject. The therapeutically effective amount of the present invention can be determined by conventional methods (e.g., modeling, dose escalation studies, or clinical trials) in combination with conventional influencing factors (e.g., route of administration, pharmacokinetics of the compound, severity and course of the disease, medical history of the subject, health status of the subject, degree of response of the subject to the drug, etc.).

As used herein, the term "inhibit", "inhibits", or "inhibiting" refers to a decrease in the baseline activity of a biological activity or process.

As used herein, the term "kit" refers to a box used to hold chemical reagents for detecting chemical Ingredients, drug residues, virus species, etc. The kit of the present invention may include (i) IN10018 or a pharmaceutically acceptable salt thereof and/or a macromolecular drug; and (ii) instructions indicating that IN10018 or a pharmaceutically acceptable salt thereof and the macromolecular drug can be used for treating a tumor in a subject, wherein the macromolecular drug is a monoclonal antibody, a bispecific antibody, or an antibody conjugate. In an embodiment, the kit includes (i) IN10018 or a pharmaceutically acceptable salt thereof; and (ii) instructions indicating that IN10018 or a pharmaceutically acceptable salt thereof and the macromolecular drug can be used for treating a tumor in a subject, wherein the macromolecular drug is a monoclonal antibody, a bispecific antibody, or an antibody conjugate. In an embodiment, the kit includes (i) a macromolecular drug; and (ii) instructions indicating that IN10018 or a pharmaceutically acceptable salt thereof and the macromolecular drug can be used for treating a tumor in a subject, wherein the macromolecular drug is a monoclonal antibody, a bispecific antibody, or an antibody conjugate. In an embodiment, the kit includes (i) IN10018 or a pharmaceutically acceptable salt thereof and the macromolecular drug; and (ii) instructions indicating that IN10018 or a pharmaceutically acceptable salt thereof and the macromolecular drug can be used for treating a tumor in a subject, wherein the macromolecular drug is a monoclonal antibody, a bispecific antibody, or an antibody conjugate.

The compounds in the kit can be contained in separate containers. Optionally, two or more compounds are contained in the same container. For example, the kit can include a first container, a second container, and a package insert, wherein the first container includes at least one dose of a drug including IN10018 or a pharmaceutically acceptable salt thereof, the second container includes at least one dose of a macromolecular drug, wherein the macromolecular drug is a monoclonal antibody, a bispecific antibody, or an antibody conjugate, and the package insert includes instructions for using the drug to treat the tumor of the subject. The first container and the second container can be the same or different shapes (e.g., vials, syringes, and bottles) and/or materials (e.g., plastic or glass). The kit can also include other materials that can assist in administering the drug, such as diluents, filters, IV bags and lines, needles, and syringes.

The exact amount of IN10018 or a pharmaceutically acceptable salt thereof and the macromolecular drug administered to a subject will depend on various factors, such as the given drug or compound, the drug Preparation, the route of administration, the type of disease, the condition, the identity of the subject or host being treated, etc., but can nevertheless be routinely determined by one skilled in the art. For example, the determination of an effective amount will also depend on the extent, severity, and type of cell proliferation. A skilled artisan will be able to determine an appropriate dosage based on these and other factors.

IN10018 or a pharmaceutically acceptable salt thereof and a macromolecular drug can be administered by a suitable route such as oral, intravenous, intramuscular or subcutaneous administration.

For example, when administered orally, the drug can be administered orally with a pharmaceutically acceptable carrier, such as an inert diluent or an assimilable edible carrier. They can be encapsulated in hard-shell or soft-shell gelatin capsules, compressed into tablets, or mixed directly with the patient's food. For example, the drug can be combined with one or more excipients and used in the form of an ingestible tablet, oral tablet, lozenge, capsule, elixir, suspension, syrup, or wafer. Tablets, lozenges, pills, capsules, etc. may further include: a binder, such as gum tragacanth, gum arabic, corn starch, or gelatin; an excipient, such as dicalcium phosphate; a disintegrant, such as corn starch, potato starch, alginic acid, etc.; a lubricant, such as magnesium stearate; or a sweetener, such as sucrose, fructose, lactose, or aspartame; or a flavoring agent.

For example, when administered intravenously or intraperitoneally by infusion or injection, a solution of the drug can be prepared in water, optionally mixed with a nontoxic surfactant.

Exemplary pharmaceutical dosage forms for injection or infusion include sterile aqueous solutions, dispersions, or sterile powders containing the active ingredient, which are suitable for the extemporaneous preparation of sterile injectable or infusible solutions or dispersions. In any case, the final dosage form should be sterile, fluid, and stable under the conditions of manufacture and storage.

The sterile injectable solution can be prepared by incorporating the desired amount of the drug into an appropriate solvent with the various other ingredients listed above as required, followed by filtered sterilization. For sterile powder used for the preparation of the sterile injectable solution, the preferred preparation methods may be vacuum drying and freeze drying techniques, which can produce a powder of the active ingredient plus any other desired ingredients that have been previously sterile filtered.

The amount of IN10018 or a pharmaceutically acceptable salt thereof and the macromolecular drug required for treatment may vary not only with the specific agent selected, but also with the route of administration, the nature of the disease being treated, and the age and condition of the patient, and may ultimately be determined at the discretion of the attending physician or clinician. However, in general, the dosage may be in the range of about 0.1 to about 50 mg/kg body weight per day.

In some embodiments, IN10018 or a pharmaceutically acceptable salt thereof is administered at a dose ranging from 5 mg/day to 100 mg/day, e.g., 20 mg /day, in adults, on a free base basis.

The macromolecular drug is administered at a dose of 1-20 mg/kg per week in adults. In a specific embodiment, trastuzumab is administered at a dose of 1-5 mg/kg per week in adults, for example, 2 mg/kg per week. In a specific embodiment, ESG-401 is administered at a dose of 2-20 mg/kg per week in adults, for example, 2 mg/kg, 3 mg/kg, 4 mg/kg, 5 mg/kg, 6 mg/kg, 7 mg/kg, 8 mg/kg, 9 mg/kg, or 10 mg/kg per week. In a specific embodiment, Sacituzumab Govitecan is administered at a dose of 2-20 mg/kg per week in adults, for example, 6 mg/kg or 10 mg/kg per week.

Technical and scientific terms used herein without specific definition have the same meaning as commonly understood by one of ordinary skill in the art to which this invention belongs.

## EXAMPLE

The following examples are provided to further describe the present disclosure. It should also be understood that these examples are only for the purpose of illustrating the present disclosure, rather than limiting the scope thereof.

The experimental methods without specific conditions in the following examples can be carried out according to the conventional conditions of this type of reaction or the conditions suggested by the manufacturer.

The experimental materials and reagents used in the following examples can be commercially available unless otherwise specified.

The abbreviations used in the examples have the following meanings:

| | |
|---|---|
| AAALAC | Association for Assessment and Accreditation of Laboratory Animal Care |
| BIW | Twice a week |
| BW | Body Weight |
| BWL | Body Weight loss |
| d | Day |
| FBS | Fetal bovine serum |
| i.v. | Intravenous injection |
| IACUC | Animal Care and Use Committee |
| L-15 | Leibovitz's L-15 medium |
| mL/kg | Milliliter/kilogram |
| mpk | Milligram/kilogram |
| mg/mL | Milligram/milliliter |
| MTV | Mean tumor volume |
| N | quantity |
| N/A | not applicable |
| p.o./PO | Intragastric administration |
| PBS | Phosphate buffer |
| DPBS | Dulbecco's phosphate buffered saline |
| P.S. | Penicillin-streptomycin dual antibody |
| QD or q.d. | Once a day |

(continued)

| | |
|---|---|
| QW | Once a week |
| RT | Room temperature |
| RTV | Relative tumor volume |
| SEM | Standard error |
| TGI | Tumor growth inhibition rate |
| TV | Tumor volume |
| w | week |
| S.C. | Subcutaneous injection |
| SPF | Specific pathogen-free |
| mm | Millimeter |
| mL | Milliliter |
| μL | Microliter |
| mg | Milligram |
| kg | Kilogram |
| °C | Degrees Celsius |
| H-Score | Histochemistry score |
| T-GFP | Green fluorescent protein-labeled trastuzumab |
| OCT | Water-soluble mixture of polyethylene glycol and polyvinyl alcohol |
| DAPI | 4',6-diamidino-2-phenylindole dihydrochloride |

**Example 1: In vivo pharmacological study of the tumor infiltration level of green fluorescent protein-labeled trastuzumab (T-GFP) in a subcutaneous xenograft tumor model of human gastric cancer NCI-N87 cells in Balb/c-nude mice after administration of IN10018**

Purpose of the experiment:

The purpose of this experiment is to evaluate the tumor infiltration level of the test article, green fluorescent protein-labeled trastuzumab (T-GFP), in a subcutaneous xenograft tumor model of human gastric cancer NCI-N87 cells in Balb/c-nude mice before and after administration of IN10018 for a period of time. Frozen sections were performed on the tumors treated with the test article T-GFP, and multiplex immunofluorescence staining was used. The expression of each protein marker was observed after whole-slide scanning, and the images was analyzed by using the HALO image analysis software.

Experimental design:

Grouping and dosing are shown in Table 1.

Table 1. Animal grouping and dosing regimens for in vivo efficacy experiments

| Group | N[1] | Test article | Dosage (mg/kg) | Parameter for dosing volume (mL/kg)[2] | Dosing route | Dosing frequency |
|---|---|---|---|---|---|---|
| 1 | 2 | Control group | N/A | 10 | PO | QD x 27 days |

(continued)

| Group | N[1] | Test article | Dosage (mg/kg) | Parameter for dosing volume (mL/kg)[2] | Dosing route | Dosing frequency |
|---|---|---|---|---|---|---|
| 2 | 2 | IN10018+T -GFP | 25+10 | 10 | PO | QD x 27 days + IV x 1 time |
| Note:<br>N: number of mice in each group;<br>Dosing volume: 10 mL/kg based on body weight of mice. If the body weight loss exceeded 15%, the dose was stopped; the dose was resumed when the body weight loss recovered to within 10%. | | | | | | |

Experimental Materials:

Mice: 7-8 weeks old female BALB/c-nude-mice were purchased from GemPharmatech Co., Ltd., Jiangsu, China. After the animals arrive, they should be housed adaptively in the experimental environment for at least 3 days before the experiment begins. The animals were housed in cages (5 animals per cage) in an SPF-grade animal room. All cages, bedding, and drinking water were sterilized before use. All experimental personnel were required to wear protective clothing and latex gloves when working in the animal room. Cages, feed, and drinking water were exchanged twice a week. The housing environment and lighting conditions were as follows:

Temperature: 20-26 °C
Humidity: 40-70%
Light cycle: 12 hours of light and 12 hours of light-free
Cages: Made of polycarbonate, 300 mm × 180 mm × 150 mm. Bedding is corncob, exchanged twice a week.
Food: The experimental animals had access to food (sterilized by irradiation, dry pelleted food) ad libitum throughout the study.
Drinking water: The experimental animals can drink sterile water ad libitum.
Cage identification: The animal information card for each cage should indicate the number of animals in the cage, sex, strain, receipt date, dosing regimen, experiment number, group, and experiment start date.
Animal identification: The experimental animals were identified with ear tags.

The information of test article is shown in Table 2.

Table 2:

| | | |
|---|---|---|
| **Name** | IN10018 | Green fluorescent protein-labeled trastuzumab |
| **Code name** | IN10018 | T-GFP |
| **Source** | Synthesized according to the method in patent WO2010058032 | BIOINTRON |
| **Batch number/ Art. Number** | Lot: CR-C18091729-B19001 | Cat: B22328001-CHO Lot: 20221126NA07 |
| **Concentration/dose** | 40 mg | 1.39 mg/ml |
| **Character** | White powder | Light green clear liquid |
| **Vehicle** | DPBS | DPBS |
| **Storage conditions** | Normal temperature | -80 °C |

The information of experimental reagent is shown in Table 3.

Table 3:

| Reagents | Brand | Art. No |
|---|---|---|
| Anhydrous ethanol | Sinopharm Chemical Reagent Co., Ltd. | 100092683 |
| Xylene | Sinopharm Chemical Reagent Co., Ltd. | 1330-20-7 |

(continued)

| Reagents | Brand | Art. No |
|---|---|---|
| Tween-20 | Sinopharm Chemical Reagent Co., Ltd. | Tween 20 |
| $H_2O_2$ | Zhongshan Golden Bridge | PV-6001 |
| Goat serum blocking solution | CWBIO | 01380/34021 |
| Primary antibody diluent | Zhongshan Golden Bridge | ZLI-9030D |
| Enzyme-labeled donkey anti-goat IgG polymer | Proteintech | SA00001-3 |
| Enzyme-labeled goat anti-rabbit IgG polymer | Zhongshan Golden Bridge | PV-6001 |
| Enzyme-labeled goat anti-mouse IgG polymer | Zhongshan Golden Bridge | PV-6001 |
| Enzyme-labeled goat anti-rat IgG polymer | Zhongshan Golden Bridge | PV-6001 |
| Hematoxylin stain | Zhuhai BaSO Biotechnology Co., Ltd | BA4041 |
| Differentiation Solution | Leagene | DH0085 |
| DAB Substrate Kit | Zhongshan Golden Bridge | ZLI-9018 |
| Fluorescent dye diluent | AKOYA | FP1498 |
| 480 Dye | AKOYA | OP-001000 |
| 520 Dye | AKOYA | OP-001001 |
| 620 Dye | AKOYA | OP-001004 |
| 650 dye | AKOYA | OP-001005 |
| 690 Dye | AKOYA | OP-001006 |
| 780 Dye | AKOYA | OP-001008 |
| 570 Dye | AKOYA | OP-001003 |
| Antifade mounting medium | Beyotime | PO131 |
| DPBS-2 | Shanghai Basalmedia Technologies Co., Ltd. | B210KJ |

The information of experimental equipment is shown in Table 4.

Table 4:

| Name | Vender | Model |
|---|---|---|
| Dehydrator | Leica | Leica ASP300S |
| Embedding Machine | Leica | HistoCore Arcadia H |
| Pathological Microtome | Leica | HistoCore BIOCUT |
| Cold Plate | Leica | HistoCore Arcadia C |
| Tissue Flattening Table | Leica | HI1210 |
| Slide Warmer | Leica | HI1220 |
| Microscope Slide | Jiangsu Ctiotest Labware Manufacturing Co., Ltd. | 80312-3161 |
| Coverslip | Nantong Mevid Life Scientific Co.,Ltd | CS01-2450 |
| Micro-wave oven | Midea | M1-L3B |
| Decolorizing Shaker | Haimen Kylin-Bell Lab Instruments Co., Ltd. | T8-1000 |
| Vortex Mixer | Haimen Kylin-Bell Lab Instruments Co., Ltd. | VORTEX5 |
| PAP Pen | ImmEdg | H-4000 |
| Brightfield Scanner | Ningbo Sunny Instruments Co., Ltd. | HS6 |
| Water purification machine | Millipore | SYNSVR000 |

(continued)

| Name | Vender | Model |
|---|---|---|
| Automated Hematoxylin Stainer | Leica | ST5020 |
| Coverslipper | Leica | CV5030 |
| Fluorescence scanner | Leica | VERSA8 |
| Microscope | OLYMPUS | CX23 |
| Fully Automated Multiplex Stainer | Leica | BOND RX |

Experimental methods and steps:

Cell culture:

Human gastric cancer cell line NCI-N87 (source: Nanjing Cobioer Biotechnology Co., Ltd., Art. No: CBP60491) was cultured in vitro as a monolayer in RPMI-1640 medium supplemented with 10% fetal bovine serum at 3°C in a 5% $CO_2$ incubator. Cells were routinely digested with trypsin-EDTA two to three times weekly for passage. When cells reached the exponential growth phase with 80-90% confluency, cells were harvested, inoculated after counted.

Cell inoculating and grouping:

0.1 mL of PBS: Matrigel=1: 1 cell suspension containing $5\times10^6$ cells was subcutaneously inoculated onto the right flank of each mouse. When tumor volume reached approximately 125.6 $mm^3$ (day 14 after cell inoculation), mice were randomly divided into groups based on tumor volume and dosed. Group information is shown in Table 1.

The preparations of the test articles and control vehicle are shown in Table 5.

Table 5:

| Name | Dosage (mg/kg) | Concentr ation (mg/ml) | Preparation method | Storage conditions and Preparation frequency |
|---|---|---|---|---|
| Control group | N/A | N/A | Aspirate an appropriate amount of DPBS. | Store at 4 °C |
| IN10018 | 25 | 2.5 | Weigh 5.5 mg of IN10018, dilute with 2.2 mL of DPBS, and mix thoroughly to prepare a clear solution. | Store at 4 °C and prepare once every 2-3 days. |
| T-GFP | 10 | 1 | Take 0.86 ml of T-GFP stock solution, add 0.24 ml of DPBS, and mix well. | Prepare before use |

Daily observation of experimental animals:

The design and any modifications to this experimental protocol were reviewed and approved by the IACUC. The use and welfare of experimental animals were carried out in accordance with AAALAC guidelines. Animal health and mortality were monitored daily. Routine examinations included observation of tumor growth and the effects of drug treatment on daily animal behavior, such as activity, food and water intake (via visual observation only), body weight change, physical signs, or other abnormalities. The number of deaths and side effects in each group was recorded based on the number of animals in each group.

Experiment termination:

Euthanasia should be performed if the animal's health continues to deteriorate, or the tumor volume exceeds 3,000 $mm^3$, or if the animal has severe illness or pain. It should notify the veterinarian, and euthanasia should be performed if the animal exhibits any of the following: significant emaciation, with a body weight loss greater than 20%; cannot freely take food or drink; or if the mean tumor volume in the control group reaches 2,000 $mm^3$, the experiment should be terminated. The animal exhibits the following clinical signs with progressive worsening should also be euthanized: piloerection, hunched posture, pale of ears, nose, eyes, or feet, rapid breathing, convulsions, persistent diarrhea,

dehydration, slowed movement, or vocalization.

Tumor measurement and excision:

The object of experimental is to examine whether tumor growth is inhibited, delayed, or cured. Tumor diameter is measured three times a week using a vernier caliper. Tumor volume is calculated using the formula: $V = 0.5 \times a \times b^2$, where a and b represent the long and short diameters of the tumor, respectively.

All animals were injected with T-GFP once through the tail vein on day 28 after continuous IN10018 treatment, that is, on day 42 after inoculation. At 6 hours post-injection, all animals were euthanized, the tumors were excised, and immersed in liquid nitrogen for approximately 10 minutes. Subsequently, the tumor samples were transferred to dry ice and immediately proceeded to the next step of research.

Frozen sections:

Tumor tissues rapidly frozen in liquid nitrogen were first subjected to a sugar immersion process according to the procedure of 20%-30%-30% (sucrose solution), then embedded with OCT to create frozen blocks. Frozen sections are prepared using a freezing microtome and mounted on adhesive slides. The sections were examined under a microscope to ensure integrity, with no significant fragmentation or folding. After completing the preparation, the slides can be left at room temperature for a period of time before directly staining, or stored in a low-temperature refrigerator.

DAPI Counterstaining and sealing the slide:

DAPI working solution (about 100-150 μl) was added to the slide dropwise, immersing the tissue area. The slide was then incubated at room temperature in a humidified chamber for 3-5 minutes. After incubation, the slide was washed with 1×TBST (or PBS) for 5 minutes, then washed with distilled water for 3 minutes. Finally, the antifade mounting medium was added dropwise to seal the slide. The coverslip was fixed with nail polish, and then the instrument was used for whole-slide scanning and subsequent data analysis.

Whole-slide scanning and quantitative analysis:

Whole-slide scanning of the entire section was performed by using a digital pathology imaging whole-slide scanning system, and the pathological image information for the entire section was obtained. The scan results were quantitatively analyzed using the pathology image analysis software Halo. The HALO Highplex FL (Indica Labs; Albuquerque, NM) analysis module of the Halo software was used. During the analysis process, the parameters corresponding to the analysis module were adjusted according to the actual conditions of the image. After determining the optimal parameters, all similar images were analyzed using the same analysis template to avoid the influence of human subjective factors. The information circled in different areas at each time represents an analysis layer, and the different staining signals of each cell within the analysis field of view are identified. At the same time, after the quantitative analysis of each layer of each image is completed, the overall information of all results will be statistically summarized and analyzed.

Analysis indicators:

The percentage of GFP-positive cell: For each defined cell type, the number of positive cells in the sample and the percentage of this type of cell in the total number of cells in the sample were statistically summarized.

H-Score: The H-Score is also known as histochemical score. By using each staining channel as a unit, the total number of positive cells in each channel of each sample and the number of cells in the weak, moderate, and positive grades was counted in the analysis result. The corresponding positive cells were counted in the three parts of the nucleus, cytoplasm, and cell membrane, respectively, and the percentage of corresponding positive cells in the overall cells and the H-Score value of each channel were calculated. The H-Score ranges from 0 to 300 and is calculated as follows: H-Score = $[1 * (\%Cells_1+) + 2 * (\%Cells_2+) + 3 * (\%Cells_3+)]$, wherein $\%Cells_{1+}$, $\%Cells_{2+}$, $\%Cells_{3+}$are the percentages of cells with weak, moderate, and positive expression levels automatically identified by the software, respectively.

Experimental results:

Image scan results

Whole-slide scanning of the entire section was performed by using a digital pathology imaging whole-slide scanning system, and the pathological image information for the entire section was obtained. The report displays

thumbnails of each image, as shown in FIG. 1.

Study on the percentage of tumor infiltration level of the Test Article T-GFP in a subcutaneous xenograft tumor model of human gastric cancer NCI-N87 cells in Balb/c-nude mice after administration of IN10018.

The experiment was conducted according to the dosing regimen. All animals were injected with T-GFP once through the tail vein on day 28 after continuous IN10018 treatment, that is, on day 42 after inoculation. At 6 hours post-injection, all animals were euthanized, the tumors were excised, frozen embedded blocks were prepared, and frozen sections were conducted. After DAPI staining, whole-slide scans were performed using a digital pathology imaging whole-slide scanning system. Quantitative analysis of the scan results was performed using the HALO Highplex FL (Indica Labs; Albuquerque, NM) analysis module of the pathology image analysis software, Halo software. During analysis, the parameters corresponding to the analysis module were adjusted according to the actual conditions of the image. After determining the optimal parameters, all similar images were analyzed using the same analysis template to avoid the influence of human subjective factors. The information circled in different areas at each time represents an analysis layer. The layer name can be customized to identify the different staining signals of individual cells within the analysis field of view. After the quantitative analysis of each layer of each image is completed, the overall information of all results will be statistically summarized. For each defined cell type, the number of positive cells in the sample and the percentage of this type of cell in the total number of cells in the sample were statistically summarized.

The total number of cells detected in the first animal of the control group was 132,264, among which the number of GFP-positive cells was 7,532, and the proportion of GFP-positive cells was 5.69%; the total number of cells detected in the second animal of the control group was 235,747, among which the number of GFP-positive cells was 11,757, and the proportion of GFP-positive cells was 4.99%. The average proportion of positive cells in the entire control group was 5.34%. The total number of cells detected in the first animal of the IN10018+T-GFP group was 104,126, among which the number of GFP-positive cells was 13,470, and the proportion of GFP-positive cells was 12.94%; the total number of cells detected in the second animal of the IN10018+T-GFP group was 182,491, among which the number of GFP-positive cells was 14,994, and the proportion of GFP-positive cells was 8.22%. The average proportion of positive cells in the entire IN10018+T- GFP group was 10.58%. See Table 6 and FIG. 2.

Table 6: Evaluation of the percentage of tumor infiltration level of the test article T-GFP in a subcutaneous xenograft tumor model of human gastric cancer NCI-N87 cells in Balb/c-nude mice after administration of IN10018

| Name | Total number of cells detected | Number of GFP-positiv e cells | The percentage of GFP-positive cells (%)$^1$ | The percentage of GFP-positive cells in each group (%)$^2$ |
|---|---|---|---|---|
| The first animal in the control group | 132264 | 7532 | 5.69 | 5.34 |
| The second animal in the control group | 235747 | 11757 | 4.99 | |
| The first animal in the IN10018+T-GFP group | 104126 | 13470 | 12.94 | 10.58 |
| The second animal in the IN10018+T-GFP group | 182491 | 14994 | 8.22 | |
| Note:<br>The percentage of GFP-positive cells = The number of GFP-positive cell/The total number of cells detected*100%;<br>The percentage of GFP-positive cells in each group is the average of the percentages of GFP-positive cells in the two animals in the group. | | | | |

H-Score study of the tumor infiltration level of the test article T-GFP in a subcutaneous xenograft tumor model of human gastric cancer NCI-N87 cells in Balb/c-nude mice after administration of IN10018

The experiment was performed according to the experimental scheme. After whole-slide scanning using a digital pathology imaging whole-slide scanning system, the HALO Highplex FL (Indica Labs; Albuquerque, NM) analysis module of the Halo software was used to count the total number of positive cells in each channel of each sample and the number of cells in the weak, moderate, and positive grades by using each staining channel as a unit. The corresponding positive cells were counted in the three parts of the nucleus, cytoplasm, and cell membrane, respectively, and the percentage of corresponding positive cells in the overall cells and the H-Score value of each channel were calculated.

The percentages of weakly positive, moderately positive, and strongly positive cells detected in the first animal of the control group were 4.87%, 0.51%, and 0.31%, respectively, and the H-Score calculated by the formula was 6.83; the percentages of weakly positive, moderately positive, and strongly positive cells detected in the second animal of the control group were 4.21%, 0.56%, and 0.22%, respectively, and the H-Score calculated by the formula was 5.98. The average H-Score of the entire control group was 6.40. The percentages of weakly positive, moderately positive, and

strongly positive cells in the first animal of the IN10018+T-GFP group were 9.83%, 1.66%, and 1.45%, respectively, and the H-Score calculated by the formula was 17.49; the percentages of weakly positive, moderately positive, and strongly positive cells in the second animal of the IN10018+T-GFP group were 5.97%, 1.30%, and 0.95%, respectively, and the H-Score calculated by the formula was 11.41. The average H-Score for the entire IN10018+T-GFP group was 14.45. See Table 7 and FIG. 3.

Table 7: H-Score evaluation of the tumor infiltration level of the test article T-GFP in a subcutaneous xenograft tumor model of human gastric cancer NCI-N87 cells in Balb/c-nude mice after administration of IN10018

| **Name** | The percentage of weakly positive cells (%)[1] | The percentage of moderately positive cells (%)[1] | The percentage of strongly positive cells (%)[1] | H-Score[2] | Average H-Score within the group[3] |
|---|---|---|---|---|---|
| The first animal in the control group | 4.87 | 0.51 | 0.31 | 6.83 | 6.40 |
| The second animal in the control group | 4.21 | 0.56 | 0.22 | 5.98 | |
| The first animal in the IN10018+T-GFP group | 9.83 | 1.66 | 1.45 | 17.49 | 14.45 |
| The second animal in the IN10018+T-GFP group | 5.97 | 1.30 | 0.95 | 11.41 | |
| Note:<br>The percentage of positive cells = The number of GFP (weak, moderate, strong) positive cell / The total number of cells detected * 100%;<br>H-Score = [1 * (%Cells$_{1+}$) + 2 * (%Cells$_{2+}$) + 3 * (%Cells$_{3+}$)], where %Cells$_{1+}$, %Cells$_{2+}$, %Cells$_{3+}$are the percentages of cells with weak, moderate, and positive expression levels automatically identified by the software, respectively;<br>The average H-Score within the group is the average of the H-Score of the two animals in the group; | | | | | |

Experimental Conclusion

In this study, we evaluated the comparison of the tumor infiltration level of the test article T-GFP in a subcutaneous xenograft model of human gastric cancer NCI-N87 cells in Balb/c-nude mice before and after administration of IN10018. After T-GFP administration for 6 hours, the tumor infiltration levels and corresponding H-Scores for each group are shown in Table 7, FIG. 1, FIG. 2, and FIG. 3.

The IN10018+T-GFP group showed a higher percentage of GFP+ positive cells and a higher H-Score than the control group after the administration of IN10018 for a period of time (27 days), compared with the percentage of tumor infiltration level and the corresponding H-Score in two mice in the blank control group. The data above suggest that IN10018 can more effectively promote drug binding to human gastric cancer cells after the administration of IN10018 for a period of time, potentially leading to better in vivo efficacy.

**Example 2: Pharmacodynamic Study of IN10018 and ESG-401 in Balb/C Nude Mice Bearing MDA-MB-468 Tumor Model**

The experimental design is shown in Table 8.

Table 8: Experimental design

| Group | n[a] | Test article | Dosage (mpk) | Dosing Volume [b] (mL/kg) | Dosing route | Dosing frequency |
|---|---|---|---|---|---|---|
| 1 | 7 | Control group | N/A | 10 | p.o. | q.d. |
| 2 | 7 | ESG-401 | 1 | 5 | i.v. | biw |
| 3 | 7 | IN10018 | 25 | 10 | p.o. | q.d. |

(continued)

| Group | n[a] | Test article | Dosage (mpk) | Dosing Volume [b] (mL/kg) | Dosing route | Dosing frequency |
|---|---|---|---|---|---|---|
| 4 | 7 | ESG-401+IN10018 | 1+25 | 5+10 | i.v.+p.o. | biw+q.d. |
| Note:<br>a. n is the number of animals in each group;<br>b. Dosing Volume: The dose is adjusted based on 10 mL/kg or 5 mL/kg of body weight. If body weight loss is >15%, the dose may be adjusted based on body weight. | | | | | | |

Experimental Materials

Mice: 6-8 weeks old female BALB/c-nude mice (body weight: 19-23 g) were purchased from the Laboratory Animal Management Department of the Shanghai Family Planning Research Institute. After the animals arrive, they should be housed adaptively in the experimental environment for at least 3 days before the experiment begins. The animals were housed in cages (3-4 animals per cage). All cages, bedding, and drinking water were sterilized before use. All experimental personnel were required to wear protective clothing and latex gloves when working in the animal room. Cages, feed, and drinking water were exchanged twice a week. The housing environment and lighting conditions were as follows:

Temperature: 18-26 °C.
Humidity: 40%-70%.
Cages: Made of polycarbonate, 300 mm × 180 mm × 150 mm in size. Bedding: corncob.
Cage Identification: The identification tag for each cage containing the following information: number of animals, sex, date of receipt, compound, study number, group number, and treatment start date.
Animal identification: The animals were identified with ear tags.

The information of test article is shown in Table 9.

Table 9:

| **Name** | IN10018 | ESG-401 |
|---|---|---|
| **Source** | Synthesized according to the method in WO2010058032 | Synthesized according to the method in WO2021225892 |
| **Concentration /dosage** | 40 mg | 10.5 mg/mL |
| **Character** | White powder | Liquid |
| **Vehicle** | DPBS | DPBS |
| **Storage conditions** | 4 °C | 4 °C |

The information of experimental reagent is shown in Table 10.

Table 10:

| **Reagents** | **Brand** | **Art. No** |
|---|---|---|
| PBS | Beyotime | 71321210824 |
| L-15 medium | Gibco | 2276800 |
| FBS | Gibco | 2120135CP |
| Double-antibody (P.S.) | Gibco | 2321126 |
| Pancreatic enzymes | Beyotime | 061522220705 |
| Matrigel | CORNING | 2208001 |
| Sodium chloride injection (Saline) | Shandong Hualu Pharmaceutical Co., Ltd. | SD22012814 |
| Natrosol 250 HX | Aladdin | H104792 |

The preparation of the compounds is shown in Table 11.

Table 11:

| Compound | Dosage (mpk) | Dosing volume (mL/kg) | Dosing concentration (mg/mL) | Preparation |
|---|---|---|---|---|
| ESG-401 | 1 | 5 | 0.2 | Take 0.038mL of ESG-401 stock solution each time, then add 1.962 mL of saline to achieve the final concentration of the solution of 0.2 mg/mL. Prepare it fresh each time. |
| IN10018 | 25 | 10 | 2.5 | Weigh a certain amount of IN10018 powder each time, then add a certain volume of 0.5% Natrosol 250 HX to achieve the final concentration of the drug of 2.5 mg/mL. The frequency of preparation is twice a week. |

Experimental methods

MDA-MB-468 cells (Beyotime) were cultured in vitro (37 °C, 0% $CO_2$) in L-15 (Gibco) medium supplemented with 10% FBS (Gibco) and 1% P.S. (Gibco). Cells were passaged 2-3 times weekly. Exponential growth phase MDA-MB-468 cells were harvested and resuspended in a suspension of PBS and Matrigel (1: 1 volume ratio of PBS to Matrigel) at a concentration of $5 \times 10^7$/mL. The prepared suspension was then used to inoculate mice. 0.2 mL of the MDA-MB-468 tumor cell suspension ($10 \times 10^6$ cells) was subcutaneously inoculated onto the right flank of each mouse. When the mean tumor volume reached approximately 160 $mm^3$, i.e., on day 7 after cell inoculation, mice were randomly divided into groups based on tumor volume. Dosing began on the same day of grouping. Detailed dosing information is shown in Table 8.

Clinical observation

All procedures related to animal handling, care, and treatment in the study were performed according to guidelines approved by the Institutional Animal Care and Use Committee (IACUC) under the guidance of the Association for Assessment and Accreditation of Laboratory Animal Care (AAALAC). During routine monitoring, animals were examined daily for tumor growth and any effects of treatment on normal behavior, such as activity, food and water consumption (via visual observation only), body weight gain/loss (measured every two days or twice a week), eye/hair changes, and any other abnormal effects specified in the protocol. Deaths and observed clinical signs were recorded based on the number of animals in each cage.

Tumor measurement

The primary endpoint of the study was to determine whether tumor growth could be delayed or cured. The experimental endpoint treatment will be performed when the mean tumor volume in the control group exceeded 2000 $mm^3$ or three weeks after dosing, whichever was shorter.

Tumor size was measured twice weekly using a vernier caliper. Tumor volume is expressed in $mm^3$ and is calculated using the formula: $V = 0.5 \times a \times b^2$, wherein a and b are the long and short diameters of the tumor, respectively. Tumor size was then used to calculate the relative tumor proliferation rate (T/C%). T/C% is calculated using the formula: $T/C\%=(T_i-T_0)/(V_i-V_0)\times 100\%$, wherein $T_i$ is the mean tumor volume of the treatment group on a certain day, $T_0$ is the mean tumor volume of the treatment group on the first day of treatment (before dosing), $V_i$ is the mean tumor volume of the control group on the day $T_i$ was measured, and $V_0$ is the mean tumor volume of the control group on the first day of treatment. TGI(%) is calculated for each group using the formula: TGI(%)=[100-T/C].

Sample collection

On day 45 after dosing, the experiment was ended for mice in groups 2 to 4 according to experimental requirements. At this point, the mice in group 1 were divided into a control group and a dosing group for additional experiments. The experiment was ended for the mice in the additional experiment on day 63 according to experimental requirements. Subsequently, the mice in the additional experiment were euthanized, and their tumors were harvested and stored in 10% formalin.

Statistical analysis

Experimental data are presented as mean and standard error of the mean (SEM), including tumor volume for each group at each time point. Statistical analysis of differences in tumor volume between groups was performed. P values were calculated using GraphPad Prism 7.0 software.

T-test was used to analyze the differences in tumor volume among the groups at each time point, and $p < 0.05$ indicated a significant difference.

Experimental results

After 29 days of dosing, all groups were discontinued for observation, as required by the experiment. On day 45 after dosing, the experiment for mice in Groups 2 to 4 were terminated, as required by the experiment. Specific grouping and dosing details are detailed in the dosing records in Table 8 of the Appendix.

The body weight and tumor volume data of mice in all groups during the experiment are as follows:

The body weight of MDA-MB-468 tumor-bearing mice was regularly monitored as an indirect measure of toxicity. Following administration, no significant decrease in the mean body weight of mice in any group was observed. Detailed changes in body weight and relative body weight of MDA-MB-468 tumor-bearing mice following administration are shown in FIG. 4 and 5.

Tumor volume

The tumor growth curve during the experiment is shown in FIG. 6. After day 45 of the experiment, the mean tumor volumes of the control group, ESG-401 group, IN10018 group, and ESG-401+IN10018 combined administration group were 414.79mm$^3$, 190.10mm$^3$, 240.14mm$^3$ and 94.48mm$^3$, respectively.

T/C(%), TGI(%) and p-value

The data of T/C and TGI at different time points during the administration period are shown in Table 12. After 45 days of administration, compared with the control group, the T/C in the ESG-401 group, the IN10018 group, and the ESG-401 + IN10018 combined administration group were 10.30%, 30.13%, and -28.00%, respectively; and the TGI were 89.70 %, 69.87 %, and 128.00 %, respectively.

The significance analysis at different time points is shown in Table 13. After 45 days of administration, ESG-401 and IN10018 alone and their combination showed significant anti-tumor effects compared with the control group.

Table 12: Tumor T/C(%) and TGI(%) at different time points

| T/C(%) Treatment days | 0 | 3 | 7 | 10 | 14 | 17 | 21 | 24 | 28 | 31 | 35 | 38 | 42 | 45 |
|---|---|---|---|---|---|---|---|---|---|---|---|---|---|---|
| Group 2: | - | 34.61 | 59.86 | 5.76 | -24.37 | -43.32 | -52.00 | -55.95 | -60.12 | -49.75 | -24.70 | -0.07 | 5.27 | 10.30 |
| Group 3: | - | 66.43 | 62.08 | 1.89 | -38.66 | -60.08 | -67.43 | -71.94 | -78.49 | -57.45 | -19.04 | 4.24 | 14.60 | 30.13 |
| Group 4: | - | 49.10 | 55.79 | -33.88 | -72.13 | -95.36 | -111.37 | -111.60 | -112.91 | -96.34 | -64.35 | -40.06 | -33.16 | -28.00 |
| TGI(%) Treatment days | 0 | 3 | 7 | 10 | 14 | 17 | 21 | 24 | 28 | 31 | 35 | 38 | 42 | 45 |
| Group 2: | - | 65.39 | 40.14 | 94.24 | 124.37 | 143.32 | 152.00 | 155.95 | 160.12 | 149.75 | 124.70 | 100.07 | 94.73 | 89.70 |
| Group 3: | - | 33.57 | 37.92 | 98.11 | 138.66 | 160.08 | 167.43 | 171.94 | 178.49 | 157.45 | 119.04 | 95.76 | 85.40 | 69.87 |
| Group 4: | - | 50.90 | 44.21 | 133.88 | 172.13 | 195.36 | 211.37 | 211.60 | 212.91 | 196.34 | 164.35 | 140.06 | 133.16 | 128.00 |

Table 13: Significance analysis of tumor volume at different time points

| P-value Treatmen t days | 0 | 3 | 7 | 10 | 14 | 17 | 21 | 24 | 28 | 31 | 35 | 38 | 42 | 45 |
|---|---|---|---|---|---|---|---|---|---|---|---|---|---|---|
| Group 2: | 0.9933 | 0.3153 | 0.3573 | 0.0397 | 0.0071 | 0.0020 | 0.0015 | 0.0010 | 0.0008 | 0.0007 | 0.0009 | 0.0007 | 0.0011 | 0.0014 |
| Group 3: | 0.9746 | 0.6402 | 0.4077 | 0.0527 | 0.0009 | 0.0001 | 0.0000 | 0.0000 | 0.0000 | 0.0000 | 0.0000 | 0.0000 | 0.0001 | 0.0012 |
| Group 4: | 0.9826 | 0.4257 | 0.3385 | 0.0025 | 0.0001 | 0.0000 | 0.0000 | 0.0000 | 0.0000 | 0.0000 | 0.0000 | 0.0000 | 0.0000 | 0.0000 |

Note: T-test is used for statistical analysis, and the p-value is calculated based on the tumor volume and compared with the control group.

2 Experimental results

The purpose of this study is to evaluate the anti-tumor efficacy of ESG-401 and IN10018 alone and their combination. During the dosing period, no significant body weight loss was observed in the mice in the dosed groups, indicating that the compounds were well-tolerated by the mice.
On day 45 of dosing, the mean tumor volumes of the control group, ESG-401 group, IN10018 group, and ESG-401 + IN10018 combined administration group were 414.79mm$^3$, 190.10mm$^3$, 240.14mm$^3$, and 94.48mm$^3$, respectively.
The T/C in the ESG-401 group, IN10018 group, and ESG-401 + IN10018 combined administration group were 10.30%, 30.13%, and -28.00%, respectively; the TGI were 89.70%, 69.87%, and 128.00%, respectively.
ESG-401, IN10018 alone and their combination showed significant anti-tumor effects ($p < 0.05$), compared with the control group.
In summary, ESG-401, IN10018 alone and two-drug combination showed significant anti-tumor effects in Balb/C nude tumor-bearing mice in the MDA-MB-468 model.

**Example 3: In vivo pharmacological study of the tumor infiltration level of ESG-401 in the MDA-MB-468 model after administration of IN10018**

The information of reagents is shown in Table 14.

Table 14

| Reagents | Vender |
|---|---|
| Anhydrous ethanol | Sinopharm Chemical Reagent Co., Ltd. |
| Xylene | Sinopharm Chemical Reagent Co., Ltd. |
| Sirius Red Stain | Shanghai Aoyuan Biotechnology Co., Ltd. |
| Hydrochloric acid | Sinopharm Chemical Reagent Co., Ltd. |
| Ammonium hydroxide | Sinopharm Chemical Reagent Co., Ltd. |
| Neutral gum | Sinopharm Chemical Reagent Co., Ltd. |
| paraffin | Sinopharm Chemical Reagent Co., Ltd. |

The information of experimental equipments is shown in Table 15.

Table 15

| Name | Vender | Model |
|---|---|---|
| Dehydrator | Zhejiang Jinhua Hisure Scientifc Co.,Ltd. | LTP-200 |
| Embedding Machine | Zhejiang Jinhua Hisure Scientifc Co.,Ltd. | ES-500 |
| Pathological Microtome | Leica Microsystems (Shanghai) Co., Ltd. | RM2235 |
| Cold Plate | Zhejiang Jinhua Hisure Scientifc Co.,Ltd. | ES-300 |
| Tissue Flattening Table | Zhejiang Jinhua Hisure Scientifc Co.,Ltd. | HS 1125 |
| Oven | Shaoxing Supo Instrument Co., Ltd. | 303-ISB |
| Slide and coverslips | Jiangsu Shitai Experimental Equipment Co., Ltd. | 10212424C |
| Upright optical microscope | NIKON, Japan | ECLIPSE CI-L |
| Imaging system | NIKON, Japan | DS-U3 |
| Scanner | 3D histech (Hungary) | Pannoramic MIDI |

Tissue paraffin embedding and sectioning experimental procedures

Sampling: The fresh mouse tumor collected in Example 2 was immersed in 10% neutral formalin for at least 24 hours. Do not freeze-store the samples at 4°C. The tumor was removed from the fixative and the target tissue areas were

trimmed smooth using a scalpel blade within a fume hood. The trimmed tissues, along with their corresponding labels, were placed into dehydration box, ensuring the specimen codes on the sample bottles were clearly marked. The tissues were briefly rinsed in a water-filled container to remove excess fixative.

Dehydration: The embedding frame was placed in the basket of a dehydrator and dehydrate using a gradient of alcohols: 75% alcohol for 2 hours - 85% alcohol for 1 hour - 90% alcohol for 1 hour - 95% alcohol for 40 minutes - anhydrous ethanol I for 30 minutes - anhydrous ethanol II for 30 minutes - ethanol-benzene for 10 minutes - xylene I for 5 minutes - xylene II for 5 minutes - wax I for 1 hour - wax II for 1 hour - wax III for 1 hour.

Embedding: The wax-soaked tissue was embedded in an embedding machine. First, the melted wax was placed in the embedding frame. Before the wax solidified, the tissue was removed from the dehydration box and placed into the embedding base mold and embedding frame according to the requirements for the embedding plane. It was then cooled in a -20°C Cold Plate. Once the wax solidifies, the wax block is removed from the embedding frame and trimmed.

Sectioning: The trimmed wax block was placed on a paraffin microtome and sectioned at a thickness of 3 μm. The sections were floated on a 42°C water bath to flatten the tissue. The tissue was picked up using a slide and baked in a 60°C oven. Once the water is dried and the wax is melted, sections were removed and stored at room temperature until ready for use.

Steps for sirius red staining experiment

Deparaffinization and rehydration of paraffin Sections: The sections were placed sequentially in xylene I for 20 minutes - xylene II for 20 minutes - anhydrous ethanol I for 5 minutes - anhydrous ethanol II for 5 minutes - 95% alcohol for 5 minutes - 90% alcohol for 5 minutes - 80% alcohol for 5 minutes - 70% alcohol for 5 minutes - washed with distilled water.

Staining with Sirius Red Stain: The sections were incubated with Sirius Red Stain for 20-30 minutes and rinsed with pure alcohol.

Dehydration and sealing: The sections were placed in 95% alcohol II for 15 minutes - anhydrous ethanol I for 10 minutes - anhydrous ethanol II for 10 minutes - xylene I for 10 minutes - xylene II for 10 minutes to dehydrate and clear. The sections were taken out of xylene, air-dried briefly, and sealed with neutral gum.

The sections were examined under a microscope. Image acquisition and analysis were performed using a scanner (3D histech, Pannoramic MIDI). Thumbnails are shown in FIG. 7.

Sirius staining results showed that the degree of fibrosis proliferation in the ESG-401 monotherapy group was similar to that in the control group, while the combination group of IN10018 and ESG-401 significantly suppressed tumor fibrosis compared to the ESG-401 monotherapy group. The binding effect of ESG-401 to tumor cells in vivo can be recognized by anti-human IgG secondary antibody. The result shows a binding staining effect between the antibody and tumor cells in the ESG-401 monotherapy group, while the combination of IN10018 and ESG-401 demonstrated a more significant binding staining effect between the antibody and tumor cells compared to the ESG-401 monotherapy group.

**Example 4: In vivo pharmacodynamic study of IN10018 and Sacituzumab Govitecan (TRODELVY) in a pancreatic cancer PC-07-0067 subcutaneous xenograft BALB/c nude mouse model**

**Experimental design**

Establishment of PDX model: The human pancreatic cancer PC-07-0067 model was initially established from a surgically resected clinical sample, which was designated as P0 after being inoculated into nude mice. Inoculation of tumor tissue from P0 into the next generation of mice is referred to as P1, and so on for subsequent serial passages in nude mice. FP3 tumors were resuscitated from P2 generation tissue, and the next generation from FP3 was designated as FP4, and so on.

Animals: BALB/c nude mice, female, 6-8 weeks old, weighing 18-22 g, 60 mice in total, purchased from Beijing Weitonglihua Experimental Animal Technology Co., Ltd.

Tumor Inoculation: A 20-30 $mm^3$ PC-07-0067 FP5 tumor tissue piece was subcutaneously inoculated on the right flank of each mouse for tumor growth. The mice were randomized for dosing when the mean tumor volume reached approximately 100-150 $mm^3$. The experimental groups and dosing regimens are shown in the table below.

The animal experimental groups and dosing regimens are shown in Table 16.

Table 16:

| Group | N[1] | Compound treatment | Dosage (mg/kg) | Dosing volume parameter (μL/g)[2] | Dosing route | Dosing frequency |
|---|---|---|---|---|---|---|
| 1 | 4 | Control group | -- | 10 | PO | QDx14 |

(continued)

| Group | N[1] | Compound treatment | Dosage (mg/kg) | Dosing volume parameter (μL/g)[2] | Dosing route | Dosing frequency |
|---|---|---|---|---|---|---|
| 2 | 4 | TRODELVY | 3 | 10 | IV | BIWx14 |
| 3 | 4 | IN10018+ TRODELVY | 12.5 + 3 | 10 | PO + IV | QDx14+BIW x14 |
| Note:<br>1. N: Number of mice in each group;<br>2. Dosing volume: 10 mL/g based on the mouse body weight. If body weight loss exceeds 15%, the dose is discontinued immediately and resumed when body weight loss returns to within 10%. | | | | | | |

Animal housing: After the animals arrive, they should be housed in the experimental environment for 3-7 days before the experiment begins. Animals were housed in an SPF-grade animal room in IVC (independent ventilation system) cages (5 animals per cage). All cages, bedding, and drinking water were sterilized before use. All experimental personnel were required to wear protective clothing and latex gloves when working in the animal room. The animal information card for each cage should indicate the number of animals in the cage, sex, strain, receipt date, dosing regimen, experiment number, group, and experiment start date. Cages, feed, and drinking water were exchanged twice a week. The housing environment and lighting conditions were as follows: temperature: 20-26 °C, humidity: 40-70%, light cycle: 12 hours of light and 12 hours of light-free.

Feed ingredients: Feed meets the standards for laboratory animal food. The maximum content of pollutants is within a controllable range and is routinely inspected by the manufacturer. High-pressure sterilized drinking water is used.

Animal grouping: Animals were weighed and tumor volumes were measured before administration, and the animals were randomly assigned to groups according to tumor volume (randomized block design).

Observation: The design and any modifications to this experimental protocol were reviewed and approved by the Institutional Animal Care and Use Committee (IACUC). The use and welfare of experimental animals were carried out in accordance with the guidelines of the Association for Assessment and Accreditation of Laboratory Animal Care (AAALAC). Animal health and mortality were monitored daily. Routine examinations included observation of tumor growth and the effects of drug treatment on daily animal behavior, such as activity, food and water intake, body weight changes (the body weight was measured twice weekly), physical signs, or other abnormalities. The number of deaths and side effects in the group was recorded based on the number of animals in each group.

Experimental indicator: The experimental indicator is to examine whether tumor growth is inhibited, delayed or cured. Tumor diameter is measured with a vernier caliper twice a week. The formula for calculating tumor volume is: $V = 0.5a \times b^2$, *a* and *b* represent the long diameter and short diameter of the tumor, respectively.

The anti-tumor efficacy of a compound is evaluated using the TGI(%) or tumor proliferation rate (T/C)(%). TGI(%) reflects the rate of tumor growth inhibition. TGI(%) is calculated as follows: TGI (%)= [1 - (mean tumor volume of a treatment group at the end of dosing - mean tumor volume of that treatment group at the start of dosing) / (mean tumor volume of the solvent control group at the end of treatment - mean tumor volume of the solvent control group at the start of treatment)] × 100%.

Tumor proliferation rate T/C (%): The calculation formula is as follows: $T/C(\%) = T_i/V_i \times 100\ \%$, wherein $V_i$ is the mean tumor volume of the solvent control group at a certain measurement, and $T_i$ is the mean tumor volume of the dosing group at the same measurement.

After the experiment, the tumor weight was measured, and the percentage of $T_{weight}$ /$C_{weight}$ was calculated. $T_{weight}$ and $C_{weight}$ represent the tumor weights of the dosing group and the solvent control group, respectively.

Termination of the experiment: Euthanasia should be performed if the animal's health continues to deteriorate, or the tumor volume exceeds 3,000 $mm^{3,}$ or if the animal has seriously ill or in pain. If exist the following situation, notify the veterinarian and euthanize should be performed:

significant emaciation, with a body weight loss greater than 20%; cannot freely take food or drink; or if the mean tumor volume in the control group reaches 2,000 $mm^3$, the experiment should be terminated. The animal exhibits the following clinical signs with progressive worsening should also be euthanized: piloerection, hunched posture, pale of ears, nose, eyes, or feet, rapid breathing, convulsions, persistent diarrhea, dehydration, slowed movement, or vocalization.

Data Analysis: T-test is used for comparisons between two groups. One-way ANOVA was used for comparisons between three or more groups. If the F-values shows significant difference, multiple comparisons were performed after the ANOVA analysis. All data were analyzed using SPSS 17.0, and $p < 0.05$ was considered statistically significant.

The compounds to be tested are shown in Table 17.

Table 17:

| Name | IN10018 | **Sacituzumab Govitecan** |
|---|---|---|
| Code name | IN10018 | TRODELVY |
| Source | Synthesized according to the method in patent WO2010058032 | Immunomedics (Lot No. S21M009A) |
| Character | White powder | Powder injection |
| Vehicle | DPBS | DPBS |
| Storage conditions | 4 °C | 4 °C |

The detailed scheme for preparing the medicine is shown in Table 18:

| Compound | Preparation method | Concentration (mg/mL) | Storage conditions |
|---|---|---|---|
| Control group (DPBS) | DPBS | -- | Normal temperature |
| IN10018 | Weigh 19.29 mg of IN10018, add 12 mL of solvent control 1 (DPBS), and dissolve under ultrasonic stirring. | 1.25 | 4°C, Protect from light |
| TRODELVY | Weigh 0.72 mg of TRODELVY, add 2.4 mL of vehicle control 1 (DPBS), and dissolve under ultrasonic stirring. | 0.3 | 4°C, Protect from light |

The tartrate salt of IN10018 was weighed in the experiment;
The medicine needs to be mixed thoroughly and gently before administration to animals.

Experimental results

After 14 days of dosing, all groups were discontinued for observation, as required by the experiment. On day 14 after dosing, the experiment was terminated and all experimental mice were sampled (for use in Example 5) according to experimental requirements. Specific grouping and dosing details are detailed in the dosing records in Table 16 of the Appendix.

The body weight and tumor volume data of mice in all groups during the experiment are as follows:
The body weight of PC-07-0067 tumor-bearing mice were regularly monitored as an indirect measure of toxicity. After dosing, the mean body weight of mice in each group did not show a significant decrease.

Tumor volume

The tumor growth curve during the experiment is shown in FIG. 8. After day 14 of the experiment, the mean tumor volumes of the control group, TRODELVY group, and TRODELVY+IN10018 combined administration group were 1512 $mm^3$, 423 $mm^3$, and 104 $mm^3$, respectively.

T/C(%), TGI(%) and p-value

After 14 days of dosing, the T/C of the TRODELVY group and the TRODELVY+IN10018 combined administration group were 27.95% and 6.86%, respectively; the TGI were 78.91 % and 102.10 %, respectively.

In summary, the combined administration of TRODELVY and IN10018 in NOD-SCID tumor-bearing mice of the PC-07-0067 model showed a stronger anti-tumor effect than the TRODELVY monotherapy group.

**Example 5: In vivo pharmacological study of the tumor infiltration degree of TRODELVY after administration of IN10018 in the PC-07-006 model**

The experimental method was the same as in Example 3. The experimental tissue samples were from Example 4. See FIG. 9 for color thumbnails. The anti-human IgG secondary antibody was able to recognize the binding of

TRODELVY to tumor cells in vivo. Results showed significant antibody-tumor cell binding in the TRODELVY monotherapy group, while the combination of IN10018 and TRODELVY demonstrated even more pronounced antibody-tumor cell binding compared to the TRODELVY monotherapy group.

**Example 6: In vivo pharmacological study of the tumor infiltration level of test article, fluorescein Cy5.5-labeled Hanquyou(汉曲优) antibody (H-Cy5.5), in a subcutaneous xenograft tumor model of human gastric cancer NCI-N87 cells in Balb/c-nude mice after administration of IN10018**

The purpose of this experiment is to evaluate the tumor infiltration level of the test article, fluorescein Cy5.5-labeled Hanquyou antibody (H-Cy5.5), before and after administration of IN10018 for a period of time (7 days) in a subcutaneous xenograft tumor model of human gastric cancer NCI-N87 cells in Balb/c-nude mice by fluorescence imaging of the tumor sites of mice administered with the test article.

Grouping and dosing are shown in Table 19.

Table 19: Animal grouping and dosing regimen for in vivo pharmacological experiments

| Group | N[1] | Test article | Dosage (mg/kg) | Dosing volume parameter (mL/kg)[2] | Dosing route | Dosing frequency |
|---|---|---|---|---|---|---|
| 1 | 2 | Control group | N/A | 10 | PO | QD x 9 days |
| 2 | 2 | IN10018 | 25 | 10 | PO | QD x 9 days |
| Note:<br>1. N: Number of mice in each group;<br>2. Dosing volume: 10 mL/kg based on body weight of mice. If the body weight loss exceeded 15%, the dose was stopped; the dose was resumed when the loss recovered to within 10%. | | | | | | |

Experimental Materials:

Mice: 6-8 weeks old female BALB/c-nude mice were purchased from Shanghai Lingchang Biotechnology Co., Ltd. After the animals arrive, they should be housed adaptively in the experimental environment for at least 3 days before the experiment begins. The animals were housed in cages (5 animals per cage) in an SPF-grade animal room. All cages, bedding, and drinking water were sterilized before use. All experimental personnel were required to wear protective clothing and latex gloves when working in the animal room. Cages, feed, and drinking water were exchanged twice a week. The housing environment and lighting conditions were as follows:

Temperature: 20-26 °C
Humidity: 40-70%
Light cycle: 12 hours of light, 12 hours of light-free
Cages: Made of polycarbonate, 300 mm × 180 mm × 150 mm. Bedding is corncob, exchanged twice a week.
Food: The experimental animals had access to food (sterilized by irradiation, dry pelleted food) ad libitum throughout the study.
Drinking water: The experimental animals can drink sterile water ad libitum.
Cage identification: The animal information card for each cage should indicate the number of animals in the cage, sex, strain, receipt date, dosing regimen, experiment number, group, and experiment start date.
Animal identification: The experimental animals were identified with ear tags.

The information of test article is shown in Table 20 below.

| | | |
|---|---|---|
| Name | IN10018 | Trastuzumab |
| Code name | IN10018 | Hanquyou, H |
| Source | Synthesized according to the method in patent WO2010058032 | Wuhan Henlius |
| Character | White powder | Powder injection |
| Vehicle | DPBS | D PBS |

(continued)

| Name | IN10018 | Trastuzumab |
|---|---|---|
| Storage conditions | 4 °C | Store at 2~8 °C protect from light |

The information of experimental reagent is shown in Table 21.

| Reagents | Brand | Art. No |
|---|---|---|
| DPBS | Shanghai Basalmedia Technologies Co., Ltd. | B210KJ |

Experimental methods and steps

Cell culture:

Human gastric cancer cell line NCI-N87 (sourced from Nanjing Cobioer Biotechnology Co., Ltd., Art. No: CBP60491) was maintained and passaged by INXMED (NANJING) CO., LTD. Cells were cultured in vitro as a monolayer in RPMI-1640 medium supplemented with 10% fetal bovine serum at 37°C in a 5% $CO_2$ incubator. Cells were routinely digested with trypsin-EDTA two to three times weekly for passage. When cells reached the exponential growth phase with 80-90% confluency, they were harvested, inoculated after counted.

Cell inoculating and grouping:

0.2 mL of PBS cell suspension containing $1\times10^7$ cells was subcutaneously inoculated onto the right flank of each mouse. When tumor volume reached approximately 200.0 $mm^3$ (day 14 after cell inoculation), mice were randomly divided into groups based on tumor volume and dosed. Group information is shown in Table 19.

Synthesis of H-Cy5.5

H- Cy5.5 was synthesized by the Institute of Biophysics, Chinese Academy of Sciences in Beijing, wherein the trastuzumab concentration was 7.44 mg/ml, i.e. 50.27 uM;
The concentration of synthesized Cy5.5 is 101 μM, and the labeling ratio of dye/protein is approximately 2.01. For in vivo mouse imaging, the recommended injection dose is 1-3 nmol Cy5.5 per animal. For this experiment, a 3 nmol Cy5.5 injection dose was used per animal.

The preparation of the test article and control vehicle is shown in Table 22.

Table 22

| Name | Dose | Concentrati on | Preparation method | Storage conditions and preparation frequency |
|---|---|---|---|---|
| Control group | N/A | N/A | Aspirate an appropriate amount of DPBS. | Store at 4°C. |
| H-Cy5.5 | 1.5 nmol H-Cy5.5 per mouse | 10 nM H-Cy5.5 per mouse | Take 450ul H-Cy5.5 stock solution, add 1.8ml DPBS, mix well, and inject 150ul intravenously into each animal | Prepare and use immediately. |
| IN10018 | 25 mg/kg | 2.5 mg/mL | Weigh 20.0 mg of IN10018, dilute with 8.0 mL of DPBS, and mix thoroughly to prepare a clear solution. | 4 °C and prepare once every 3-4 days. |

Daily observation of experimental animals

The design and any modifications to this experimental protocol were reviewed and approved by the Institutional Animal Care and Use Committee (IACUC). The use and welfare of experimental animals were carried out in accordance with the guidelines of the Association for Assessment and Accreditation of Laboratory Animal Care (AAALAC). Animal health and mortality were monitored daily. Routine examinations included observation of tumor growth and the effects of drug treatment on daily animal behavior, such as activity, food and water intake (via visual observation only), body weight change, physical signs, or other abnormalities. The number of deaths and side effects in the group was recorded based on

the number of animals in each group.

Experiment termination

Euthanasia should be performed if the animal's health continues to deteriorate, or the tumor volume exceeds 3,000 $mm^3$, or if the animal has severe illness or pain. It should notify the veterinarian, and euthanasia should be performed if the animal exhibits any of the following: significant emaciation, with a body weight loss greater than 20%; cannot freely take food or drink; or if the mean tumor volume in the control group reaches 2,000 $mm^3$, the experiment should be terminated. The animal exhibits the following clinical signs with progressive worsening should also be euthanized: piloerection, hunched posture, pale of ears, nose, eyes, or feet, rapid breathing, convulsions, persistent diarrhea, dehydration, slowed movement, or vocalization.

Tumor measurement

The object of experimental is to examine whether tumor growth is inhibited, delayed, or cured. Tumor diameter is measured three times a week using a vernier caliper. Tumor volume is calculated using the formula: $V = 0.5 \times a \times b^2$, where a and b represent the long and short diameters of the tumor, respectively.

Tumor imaging

On day 7 after administration of IN10018, the G1 control group and the G2 IN10018 25mg/kg group were injected with H-Cy5.5 via the tail vein. The animals were imaged using the IVIS® Lumina III Small Animal In vivo Imaging System at 6h, 24h, and 48h after injection to detect their fluorescence intensity.

Experimental indicators

Mouse fluorescence signal value: In vivo imaging was performed on the mice, the entire trunk was gated, and collect the total fluorescence intensity (Total Radiant Efficiency) of the entire trunk.

Average fluorescence signal value of the tumor site: In vivo imaging was performed on the mice, and the tumor site was clearly gated under white light field of view. The average fluorescence intensity (Avg Radiant Efficiency) of the tumor within the gate was collected.

Statistical analysis

Statistical analysis was performed using Prism Graphpad software at the end of the experiment. Overall comparisons of average fluorescence intensity at different time points were performed using two-way ANOVA with Fisher's LSD test, and $P < 0.05$ was considered a significant difference.

Experimental results

On day 14 after tumor inoculation, 14 animals with appropriate tumor volumes were randomly divided into groups based on tumor volumes. The mean tumor volume was approximately 200 $mm^3$, and this day was designated as day 0 of dosing. Group G2 began receiving IN10018 25 mg/kg as planned. FIG. 10 shows the data for grouping and dosing on 14 days after inoculation. The mean tumor volume in the G1 control group was $200.7 \pm 30.8$ $mm^3$, and the mean tumor volume in the G2 IN10018 25 mg/kg group was $200.0 \pm 33.4$ $mm^3$ (mean $\pm$ SEM).

**Study on the total fluorescence intensity of the test article H-Cy5.5 in the subcutaneous xenograft tumor model of human gastric cancer NCI-N87 cells in Balb/c-nude mice 6 hours after of injection**

The experiment was conducted according to the dosing regimen. 150 μl of H-Cy5.5 was injected into all animals via the tail vein on day 7 after continuous IN10018 treatment, i.e., day 21 after inoculation. Six hours after the injection, the animals were imaged using the IVIS® Lumina III Small Animal In Vivo Imaging System to detect the total fluorescence intensity of their trunk.

Six hours after tail vein injection of H-Cy5.5, mice in both groups were imaged in vivo. The trunk area of mice was gated, and total fluorescence intensity (Total Radiant Efficiency) was measured. Several animals not injected with H-Cy5.5 were also imaged as negative controls. The total fluorescence intensity of the G1 control group was 2.86E+11$\pm$1.08E+11, while that of the G2 IN10018 25mg/kg group was 2.88E+11$\pm$1.09E+11, and that of the negative control group was 2.04E+10$\pm$1.18E+10. Compared with the negative control group based on the aggregate total fluorescence intensity

data, p-values for the G1 control group and the G2 IN10018 25mg/kg group were p = 0.0167 and p = 0.0167, respectively. G1 control group, compared with the G2 IN10018 25 mg/kg treatment group, based on the aggregate total fluorescence intensity data showed a p value of 0.3176. See Table 23 and FIG. 11 and FIG. 12 for details.

Table 23: Evaluation of the total fluorescence intensity of the test article H-Cy5.5 in the subcutaneous xenograft tumor model of human gastric cancer NCI-N87 cells in Balb/c-nude mice 6 hours after injection

| Name | Total fluorescence intensity[1] | P-value[2] | P-value[3] |
|---|---|---|---|
| Negative control group | 2.04E+10±1.18E+10 | / | / |
| G1 control group | 2.86E+11±1.08E+11 | 0.0167 * | 0.3176 |
| G2 IN10018 group | 2.88E+11±1.09E+11 | 0.0167 * | |
| Note:<br>1. mean±SEM;<br>2.*: p < 0.05, t-test, Mann-Whitney test, vs. negative control group;<br>3. t-test, Mann-Whitney test, G1 control group vs. G2 IN10018 25 mg/kg group; | | | |

**Study on the tumor infiltration level of the test article H-Cy5.5 in the subcutaneous xenograft tumor model of human gastric cancer NCI-N87 cells in Balb/c-nude mice at 6 hours, 24 hours and 48 hours after injection**

The experiment was conducted according to the experimental scheme. H-Cy5.5 was injected into all animals via the tail vein on day 7 after continuous IN10018 treatment, i.e., day 21 after inoculation. At 6 hours, 24 hours and 48 hours after injection, the animals were imaged using the IVIS® Lumina III Small Animal In vivo Imaging System to detect the average fluorescence intensity of their tumor sites. The tumor sites of the mice were clearly gated under white light field of view, and the average fluorescence intensity (Avg Radiant Efficiency) of the tumor within the gate was collected.

At 6 hours after H-Cy5.5 injection, the average fluorescence intensity of the tumor site in the G1 control group was 3.31E+09±1.25E+09, while the average fluorescence intensity of the tumor site in the G2 IN10018 25 mg/kg treatment group was 4.02E+09±1.52E+09. At 24 hours after H-Cy5.5 injection, the average fluorescence intensity of the tumor site in the G1 control group was 5.31E+09±2.01E+09, while the average fluorescence intensity of the tumor site in the G2 IN10018 25 mg/kg treatment group was 6.41E+09±2.42E+09. At 48 hours after H-Cy5.5 injection, the average fluorescence intensity of the tumor site in the G1 control group was 6.61E+09±2.50E+09, while the average fluorescence intensity of the tumor site in the G2 IN10018 25 mg/kg treatment group was 8.04E+09±3.04E+09. Statistical analysis was performed for the G1 control group and the G2 IN10018 25 mg/kg treatment group based on the average fluorescence intensity at each time point. The p-value for both groups was p = 0.0148 at 48 hours after injection. See Table 24 and FIG. 13 and FIG. 14 for details.

Table 24: Evaluation of average fluorescence intensity of the tumor site of the test article H-Cy5.5 in the subcutaneous xenograft tumor model of human gastric cancer NCI-N87 cells in Balb/c-nude mice at 6h, 24h and 48h after injection

| Name | 6h[1] | 24h[1] | 48h[1] | p-value[2] |
|---|---|---|---|---|
| G1 control group | 3.31E+09±1.25E+09 | 5.31E+09+2.01E+09 | 6.61E+09±2.50E+09 | 0.0148 * |
| G2 IN10018 group | 4.02E+09±1.52E+09 | 6.41E+09±2.42E+09 | 8.04E+09±3.04E+09 | |
| Note:<br>1. The average fluorescence intensity of the tumor site in each group at 6h, 24h and 48h, mean ± SEM;<br>2.*: p < 0.05, G1 control group vs. G2 IN10018 25 mg/kg group, Two-way ANOVA; | | | | |

Experimental Conclusion

In this study, we evaluated the tumor infiltration level of the test article H-Cy5.5 in a subcutaneous xenograft model of human gastric cancer NCI-N87 cells in Balb/c-nude mice before and after administration of IN10018. The tumor infiltration level and corresponding fluorescence intensity were measured at 6, 24, and 48 hours after H-Cy5.5 administration. The total fluorescence intensity of the animals at 6 hours after administration showed that the average total fluorescence intensity was consistent across groups after dosing the same dose of H-Cy5.5, indicating that the injection was successful. The average fluorescence intensity values of the animal tumor sites at 6, 24, and 48 hours after administration indicated that H-Cy5.5 at the tumor sites shows an enrichment trend over time. Furthermore, the average fluorescence intensity values of the G2 IN10018 25 mg/kg group were consistently higher than those of the G1 control

group, with a statistically significant difference at 48 hours. The data above suggest that administration of IN10018 can promote the binding of the drug to human gastric cancer NCI-N87 cells more effectively, potentially leading to better efficacy in vivo.

**Example 7: In vivo anti-tumor efficacy study of Enhertu (trastuzumab) in combination with IN10018 in a subcutaneous xenograft tumor model of BALB/c-nude mice inoculated with a mixture of human gastric cancer NCI-N87 cells and mouse embryonic fibroblasts NIH-3T3 cells**

Experimental design

Grouping and dosing are shown in Table 25

Table 25: Animal groups and dosing regimens for in vivo efficacy experiments

| Grou p | N[1] | Test drug | Dosage (mg/kg) | Dosing volume parameter (mL/kg)[2] | Dosing route | Dosing frequency |
|---|---|---|---|---|---|---|
| 1 | 6 | Control group | N/A | 10 | i.p. | QW x 5W |
| 2 | 6 | Enhertu [3] | 3 | 10 | i.p. | Day 7, Day 28 |
| 3 | 6 | IN10018[4] | 25 | 10 | PO | QD x 5W |
| 4 | 6 | Enhertu[3] + IN10018[4] | 3+25 | 10 | i.p. + PO | Day 7, 28 + QD x 5W |
| Note:<br>1. N: number of mice in each group;<br>2. Dosing volume: 10 mL/kg based on mice body weight. If the body weight drops by more than 15%, stop dosing. Resume dosing when the body weight recovers to 10% of the decrease.<br>3. Enhertu was administered starting on the day 7 after grouping and dosing, and was only administrated twice, on the day 7 and day 28 after grouping and dosing;<br>4. IN10018 was administered starting on the day of grouping and dosing; | | | | | | |

The experimental animals, breeding environment and DPBS source were the same as those in Example 6. The test sample information is shown in Table 26.

| | | |
|---|---|---|
| Name | IN10018 | Enhertu, trastuzumab for injection |
| Code name | IN10018 | Enhertu |
| Source | Synthesized according to the method in patent WO2010058032 | Commercially available |
| Character | White powder | White to yellowish white freeze-dried powder |
| Solvent | DPBS | DPBS |
| Storage conditions | 4°C | 4 °C |

Experimental methods and steps

Cell culture

Human gastric cancer cell line NCI-N87 (source: Nanjing Cobioer Biotechnology Co., Ltd., Art. No: CBP60491) was maintained and passaged by INXMED (NANJING) CO., LTD. Cells were cultured as monolayers in RPMI-1640 medium supplemented with 10% fetal bovine serum at 37°C in a 5% $CO_2$ incubator. Cells were routinely digested and passaged two to three times weekly using trypsin-EDTA.

Mouse embryonic fibroblast NIH-3T3 cells (Nanjing Cobioer Biotechnology Co., Ltd., Art. No: CBP60317) were maintained and passaged by INXMED (NANJING) CO., LTD. Cells were cultured as monolayers in RPMI-1640 medium supplemented with 10% fetal calf serum at 37°C in a 5% CO2 incubator. Cells were routinely digested and passaged two to

three times a week using trypsin-EDTA. When both cell lines reached the exponential growth phase and a confluence of 80%-90%, cells were harvested, counted, and inoculated.

Cell inoculation and grouping

For mice inoculated with a mixture of NCI-N87 and NIH-3T3 cells, 0.2 mL of a mixed cell suspension containing $10\times10^6$ NCI-N87 cells and $5\times10^5$ NIH-3T3 cells was subcutaneously inoculated into the right back of each mouse. On day 8 after inoculation, when tumor volumes reached approximately 154 $mm^3$, mice inoculated with a mixture of NCI-N87 and NIH-3T3 cells were randomly divided into groups based on tumor volume and dosed. Group information is provided in Table 25.

The preparation of test articles and control solvents is shown in Table 27.

| Name | Dosage (mg/kg) | Concentration (mg/ml) | Preparation method | Storage & Preparation |
|---|---|---|---|---|
| Control group | N/A | N/A | Aspirate a certain volume of DPBS. | Store at 4°C. |
| Enhertu | 3 | 0.3 | 100mg/bottle of Enhertu in 5ml of DPBS to formulate a stock solution. Aliquot and freeze at -20°C. Take out one aliquot for each subsequent use, thaw and prepare the solution. Store at 4 °C after preparation. Do not return to -20°C. Pipette 87.5ul of Enhertu solution, add 5.743ml of DPBS, and mix well. | Freshly prepare just before use. |
| IN 10018 | 25 | 2.5 | Weigh 36.0 mg of IN10018, add 14.4 ml of DPBS, and mix thoroughly. | Store at 4°C and prepare once every 3-4 days. |

Daily observation of experimental animals

The development of this experimental protocol and any modifications were evaluated and approved by the Institutional Animal Care and Use Committee (IACUC) before implementation. The use and welfare of experimental animals were conducted in accordance with the guidelines of the Association for Assessment and Accreditation of Laboratory Animal Care (AAALAC). Animal health and mortality were monitored daily. Routine examinations included observation of tumor growth and the effects of drug treatment on daily animal behavior, such as activity, food and water intake (visual observation only), body weight change, physical signs, or other abnormalities. Group deaths and adverse reactions were recorded based on the number of animals in each group.

Termination of experiment

Euthanasia should be performed if the animal's health continues to deteriorate, the tumor volume exceeds 3,000 $mm^3$, or if the animal shows signs of severe illness or pain. Notify the veterinarian and euthanasia should be performed if the animal exhibits any of the following: significant emaciation, with a body weight loss greater than 20%; lack of free access to food and water; or if the average tumor volume in the control group reaches 2,000 $mm^3$. The experiment should be terminated if the animal exhibits the following clinical signs and continues to deteriorate: piloerection, arched back, pale ears, nose, eyes, or feet, rapid breathing, convulsions, persistent diarrhea, dehydration, slowed movement, or vocalization.

Tumor measurement

The experimental indicator is to examine whether tumor growth is inhibited, delayed, or cured. Tumor diameter is measured three times a week using a vernier caliper. Tumor volume is calculated using the formula: $V = 0.5 \times a \times b^2$, where a and b represent the major and minor diameters of the tumor, respectively.

The tumor inhibition efficacy of a compound was evaluated using the TGI (%), which reflects the rate of tumor growth inhibition. The TGI (%) was calculated based on the tumor volume on the first day after grouping using the following formula: TGI (%) = [1 - (average tumor volume of a given treatment group - average tumor volume of that treatment group at the start of treatment) / (average tumor volume of the vehicle control group - average tumor volume of the vehicle control

group at the start of treatment)] × 100%.

Statistical analysis

Statistical analysis was performed using Prism Graphpad software based on tumor volume at the end of the study. Multiple group comparisons were performed using two-way ANOVA and Fisher 's LSD test. $P < 0.05$ was considered significant.

Experimental results

After cell inoculation, tumor growth was observed daily. On day 8 after inoculation, patients were grouped and dosed based on tumor volume (designated as Day 0). The average tumor volume at entry was approximately 154 $mm^3$. All medications, except for Enhertu (3 mg/kg), were administered starting on Day 0. Enhertu (3 mg/kg) was administered twice in all groups, starting on Day 7 and on Day 28. Due to tumor burden, all groups were euthanized on Day 43 after inoculation, 35 days after grouping and dosing, the experiment was concluded.

On day 35 after grouping and dosing, the tumor volume in the control group was 1523.7±173.3 $mm^3$; the tumor volume in the Enhertu (3 mg/kg) treatment group was 1575.2±547.4 $mm^3$; the tumor volume in the IN10018 (25 mg/kg) treatment group was 976.8±171.2 $mm^3$; and the tumor volume in the Enhertu+IN10018 (3+25 mg/kg) combination treatment group was 626±209.8 $mm^3$;

Comparing the overall tumor volume with the control group, the tumor inhibition rates (TGI) for the Enhertu (3 mg/kg) and IN10018 (25 mg/kg) monotherapy groups were -3.8 % (p = 0.7634) and 40.0 % (p = 0.0015), respectively. The TGI for the Enhertu + IN10018 (3+25 mg/kg) two-drug combination therapy group was 65.6 % (p < 0.0001). Comparing the overall tumor volume-related effects of Enhertu (3 mg/kg) monotherapy groups, IN10018 (25 mg/kg), and the Enhertu + IN10018 (3 + 25 mg/kg) two-drug combination therapy groups, the p-values were p < 0.0001 and p = 0.0921, respectively. See Table 28 for details. The tumor volumes of each dose group at different time periods are shown in FIG. 15.

Table 28: Evaluation of the tumor inhibitory effect of the test article on the BALB/c-nude mouse transplant tumor model of NCI-N87 & NIH-3T3 cells (based on data on day 35 after grouping and dosing)

| Group | Tumor volume on day 0 ($mm^3$)[1] | Tumor volume on day 35 ($mm^3$)[1] | TGI (%)[2] | P value[3] | P value[4] |
|---|---|---|---|---|---|
| Control group | 154.6±8.8 | 1523.7±173.3 | / | / | / |
| Enhertu | 154.5±10.6 | 1575.2±547.4 | -3.8 | 0.7634 | <0.0001 **** |
| IN10018 | 154.6±8.6 | 976.8±171.2 | 40.0 | 0.0015 ** | 0.0921 |
| Enhertu + IN10018 | 154.8±8.9 | 626±209.8 | 65.6 | <0.0001 **** | / |

Note:
1. Calculated according to the number of days after grouping and dosing, the data are mean ± standard error (mean ± SEM);
2. TGI (%) = [1-(T35-T0)/ (V35-V0)] ×100%;
3. **: p<0.01, ****: p<0.0001, vs. control group, Two-way ANOVA, Fisher's LSD test;
4. ****: p<0.0001, vs. Enhertu+IN10018 (3+25 mg/kg) , Two-way ANOVA, Fisher's LSD test;

The experiment was carried out according to the dosing regimen. During the experiment, the animals' activities such as feeding and drinking were observed every day, and the animal body weights were recorded three times a week. For the co-inoculated NCI-N87 human gastric cancer cells and NIH-3T3 mouse embryonic fibroblasts, on day 35 after grouping and dosing, the average body weight of the control group increased from 18.5 g on Day 0 to 22.0 g, with a body weight gain of 18.4%. The average body weight of the Enhertu (3 mg/kg) and IN10018 (25 mg/kg) monotherapy groups increased from 17.9 g and 19.2 g on Day 0 to 20.9 g and 21.5 g on Day 35, respectively, with body weight change rates of 16.9% and 11.6%, respectively. The average body weight of the Enhertu + IN10018 (3 + 25 mg/kg) combination therapy group increased from 18.9 g on Day 0 to 20.7 g on Day 35, with a body weight change rate of 9.5%. Throughout the dosing period, animals in all groups maintained good health and showed no significant body weight loss. See Table 29 for details. The body weight changes and change rates of each dosage group at different time periods are shown in FIG. 16 and FIG. 17.

Table 29: Evaluation of Body Weight Changes of the Test Substances in the BALB/c-nude Mouse Xenograft Model of NCI-N87 & NIH-3T3 Cells (Based on Data on Day 35 After Grouping and Dosing)

| Group | Animal survival [1] | Weight on day 0 (g)[2] | Weight on day 35 (g)[2] | Body weight change rate (%)[3] |
|---|---|---|---|---|
| Control group | 6/6 | 18.5±0.4 | 22.0±0.5 | 18.4 |
| Enhertu | 6/6 | 17.9±0.3 | 20.9±0.6 | 16.9 |
| IN10018 | 6/6 | 19.2±0.5 | 21.5±0.4 | 11.6 |
| Enhertu + IN10018 | 6/6 | 18.9±0.4 | 20.7±0.5 | 9.5 |

Note:
1. Calculate the number of animal survival on day 0/the number of animal survival on day 35 according to the number of days after grouping and dosing.
2. Data are mean ± standard error (mean ± SEM) ;
3. Weight change rate = $(W_{35}-W_0) / W_0 * 100\%$;

Experimental Conclusion

In this study, we evaluated the in vivo efficacy of Enhertu in combination with IN10018 in a subcutaneous xenograft tumor model in BALB/c-nude mice inoculated with a mixture of NCI-N87 & NIH-3T3 human gastric cancer cells and mouse embryonic fibroblasts. Compared with the blank control group, in the experimental animal group inoculated with a mixture of NCI-N87 & NIH-3T3 human gastric cancer cells and mouse embryonic fibroblasts, both the IN10018 (25 mg/kg) monotherapy group and the Enhertu+IN10018 (3+25 mg/kg) two-drug combination therapy group showed significant tumor growth inhibition, with statistical differences compared with the control group. In addition, the Enhertu+IN10018 (3+25 mg/kg) two-drug combination therapy group had smaller tumor volume and better tumor inhibition rate than the monotherapy group throughout the experimental cycle. The above data indicate that the combination of Enhertu and IN10018 can have a better therapeutic effect than the monotherapy group.

Taking the entire dosing cycle into consideration, the animals' body weight changes were good during the 35-day dosing cycle, and no abnormalities were found in their activities, drinking water, feeding, and mental state throughout the dosing cycle, indicating that the animals tolerated Enhertu (3 mg/kg) and IN10018 (25 mg/kg) monotherapy and combination therapy.

**Example 8: Evaluation of the anti-tumor effect of IN10018 in the PC-07-0041 human pancreatic cancer PDX model in female BALB/c nude mice**

Experimental Design

Grouping and Dosing See Table below

Table 30: Grouping and dosing regimens of experimental animals for in vivo efficacy experiments

| Grou p | N[1] | Test drug | Dosage (mg/kg) | Dosing volume parameters (mL/kg)[2] | Dosing route | Dosing frequency |
|---|---|---|---|---|---|---|
| 1 | 5 | control group | - | 10 | PO | QDx29 |
| 2 | 5 | IN10018 | 25 | 10 | PO | QDx29 |
| 3 | 5 | ESG-401 | 1/3[3] | 10 | IV | BIWx10 |
| 4 | 5 | IN10018 + ESG-401 | 25 + 1/3[3] | 10 + 10 | PO + IV | QDx29+BIWx10 |

Note:
1. N: Number of mice in each group;
2. Dosage: Adjust the dosage 10μL/g according to body weight;
3. The initial dose was 1, if it is ineffective, then the dose is increased to 3.

Experimental animals: purchased from Zhejiang Weitonglihua Experimental Animal Technology Co., Ltd., other

information is the same as Example 6.

The rearing environment and DPBS source were the same as those in Example 6.

The test sample information is shown in Table 9.

Experimental methods and steps

Establishment of PDX model:

Mice were inoculated with PC-07-0041 human pancreatic cancer tumor cells, which were established from surgically resected patient tissue. Passage 0 was defined as (P0). The next generation following P0 tumor inoculation was defined as Passage 1 (P1), and so on during serial Inoculation in mice. FP4 tumor tissue was used in this study.

Cell inoculation and grouping

Each mouse was inoculated subcutaneously with a PC-07-0041 FP4 tumor piece (approximately 30 $mm^3$) on the right flank for tumor development. Treatment was started 19 days after tumor inoculation, when the average tumor size reached approximately 119 $mm^3$. Animals were randomly assigned to groups based on tumor volume using Excel-based randomization software. Each group consisted of five tumor-bearing mice. Test articles were administered to mice according to the pre-determined schedule outlined in the experimental design table.

The preparation of the test article and control solvent is shown in Table 31.

| Name | Concentrati on(mg/ml) | Preparation method | Storage & Preparation |
|---|---|---|---|
| Control group | N/A | Aspirate a certain volume of DPBS. | Store at 4°C. |
| ESG-401 | 0.1 | Dilute 0.03 mL of 10.5 mg/mL ESG-401 with 3.12 mL of DPBS to a final concentration of 0.1 mg/mL and mix thoroughly. | Freshly prepare just before use. |
| | 0.3 | Dilute 0.08 mL of 10.5 mg/mL ESG-401 with 2.72 mL of DPBS to a final concentration of 0.3 mg/mL and mix thoroughly. | Freshly prepare just before use. |
| IN 10018 | 2.5 | Weigh 19.82 mg of IN10018, add 6 ml of DPBS, and mix well. | Store at 4°C and prepare once every 3-4 days. |
| Note: The tartrate salt of IN10018 was weighed in the experiment; | | | |

Daily observation of experimental animals

The development of this experimental protocol and any modifications were evaluated and approved by the Institutional Animal Care and Use Committee (IACUC) before implementation. The use and welfare of experimental animals were conducted in accordance with the guidelines of the Association for Assessment and Accreditation of Laboratory Animal Care (AAALAC). Animal health and mortality were monitored daily. Routine examinations included observation of tumor growth and the effects of drug treatment on daily animal behavior, such as activity, food and water intake (visual observation only), body weight change, physical signs, or other abnormalities. Group deaths and adverse reactions were recorded based on the number of animals in each group.

Tumor Measurements and Experimental Indicators

Experimental indicators assess whether tumor growth is inhibited, delayed, or cured. Tumor diameter is measured twice weekly using a vernier caliper. Tumor volume is calculated using the formula: $V = 0.5 \times a \times b^2$, where a and b represent the major and minor diameters of the tumor, respectively.

Tumor size was then used for the calculation of TGI (%) and T/C (%) values.

The TGI calculation formula for each group is: $TGI(\%)=[1-(Ti-T0)/(Vi-V0)]\times 100\%$;
Ti is the mean tumor volume of the treatment group on a given day, T0 is the mean tumor volume of the treatment group on the day treatment started, Vi is the mean tumor volume of the vehicle control group on the same day as Ti, and V0 is the mean cancer volume of the vehicle group on the day treatment started.

The T/C value (percentage) is an indicator of antitumor effectiveness; T and C are the mean volumes of the treated and control groups, respectively, on a given day.

Tumor weights were measured at the end of the study. $T/C_{weight}$ values (percentages) were calculated using the formula: $T/C_{weight}\%=T_{weight}/C_{weight} \times 100\%$, wherein, $T_{weight}$ and $C_{weight}$ are the average tumor weights of the treatment group and the vehicle control group, respectively.

Statistical analysis

Tumor volumes and weights were compared between groups using one-way analysis of variance. Because a significant F statistic (the ratio of the treatment variance to the error variance) was obtained, the Games-Howell test was used for comparisons between groups. All data were analyzed using SPSS 29.0, and $p < 0.05$ was considered statistically significant.

Experimental results

Animal body weights were monitored regularly as an indicator of toxicity. No mortality or morbidity was observed. None of the mice showed significant body weight loss. Body weight changes in the different treatment groups are shown in FIG. 18.

Tumor growth inhibition by IN10018 and ESG-401 in the PC-07-0041 PDX model was calculated based on tumor volume measurements on day 28, as shown in Tables 32, 33 and FIG. 19.

Table 32: Average tumor volume at different times

| Days | Tumor volume ($mm^3$)[1] | | | |
|---|---|---|---|---|
| | **Control group** | **IN10018 25 mg/kg** | **ESG-401 1/3 mg/kg** | **IN10018 + ESG-401 25 mg/kg + 1/3 mg/kg** |
| 0 | 119 ± 11 | 119 ± 11 | 119 ± 8 | 119 ± 8 |
| 3 | 179 ± 26 | 165 ± 18 | 152 ± 19 | 163 ± 22 |
| 7 | 265 ± 37 | 250 ± 20 | 243 ± 37 | 199 ± 38 |
| 10 | 342 ± 49 | 320 ± 30 | 334 ± 56 | 263 ± 60 |
| 14 | 447 ± 63 | 522 ± 58 | 463 ± 82 | 315 ± 90 |
| 17 | 552 ± 66 | 641 ± 80 | 541 ± 100 | 293 ± 93 |
| 21 | 710 ± 116 | 820 ± 120 | 535 ± 96 | 256 ± 90 |
| 24 | 825 ± 126 | 1,060 ± 141 | 557 ± 82 | 232 ± 83 |
| 28 | 944 ± 124 | 1,252 ± 178 | 510 ± 57 | 197 ± 63 |
| Note: 1. Mean ± SEM | | | | |

Table 33:

| Treatment group | Tumor volume ($mm^3$) Day 28 | T/C (%) | TGI (%) | *p value* |
|---|---|---|---|---|
| Control group | 944 ± 124 | - | - | - |
| IN10018, 25 mg/kg | 1,252 ± 178 | 132.65 | -37.38 | 0.525 |
| ESG-401, 1/3 mg/kg[e] | 510 ± 57 | 54.00 | 52.60 | 0.075 |
| IN10018, 25 mg/kg +ESG-401, 1/3 mg/kg | 197 ± 63 | 20.83 | 90.55 | 0.007 |

The tumor weights of tumor-bearing mice in different treatment groups are shown in Table 34.

Table 34:

| Treatment group | Tumor weight (g) Day 29 | $T/C_{weight}$ (%) | *P value* |
|---|---|---|---|
| Control group | 0.848 ± 0.255 | -- | -- |
| IN10018, 25 mg/kg | 1.184 ± 0.391 | 139.59 | 0.433 |
| ESG-401, 1/3 mg/kg[d] | 0.416 ± 0.103 | 49.00 | 0.056 |
| IN10018, 25 mg/kg + ESG-401, 1/3 mg/kg | 0.169 ± 0.133 | 19.97 | 0.007 |

In this study, the efficacy of IN10018 and ESG-401 in the PC-07-0041 human pancreatic xenograft model was evaluated in female BALB/c nude mice.

The average tumor size of control mice reached 944 $mm^3$ on day 28 after the start of treatment. Compared with the control group, IN10018+ESG-401 (25 mg/kg, QD + 1/3 mg/kg, BIW) treatment produced significant antitumor activity; The mean tumor size was 197 $mm^3$ (T/C = 20.83%, TGI = 90.55%, p = 0.007). IN10018 (25 mg/kg, QD) and ESG-401 (1/3 mg/kg, BIW) treatment did not produce significant antitumor activity; The mean tumor size was 197$mm^3$ (T/C=20.83% , TGI=90.55%, p=0.007). The mean tumor sizes were 1252 $mm^3$ (T/C = 132.65%, TGI = -37.38%, p = 0.525) and 510 $mm^3$ (T/C = 54.00%, TGI = 52.60%, p = 0.075), respectively. The results of tumor weight were basically consistent with those of tumor volume.

In this study, the test compounds IN10018 and ESG-401 were well tolerated by tumor-bearing mice at the dose levels indicated. No significant weight loss was observed in any treatment group.

**Example 9: In vivo efficacy evaluation of the test drug IN10018 as a single drug or in combination with other drugs in the human rectal cancer xenograft model LD1-0038-361855**

Experimental animals: NU/NU mice, female, weighing 18 to 21 g, purchased from Beijing Weitonglihua Laboratory Animal Technology Co., Ltd. and housed in the same environment as in Example 6.

Human rectal cancer tumor tissue (case model number: LD1-0038-361855) was passaged to FP5+2 and used in this efficacy study.

The test sample information is shown in Table 26.

The tumor masses of human rectal cancer LD1-0038-361855 in vivo transplanted tumors were cut into pieces of approximately 3 mm × 3 mm × 3 mm (approximately 45 to 60 mg) and inoculated subcutaneously into NU/NU mice. The mice were observed after inoculation and the growth of the tumor was monitored. On the 23rd day after inoculation, when the average tumor volume of the tumor-bearing mice reached 156.69 $mm^3$, grouping and dosing were performed. The day of grouping and dosing was defined as day 0. The specific grouping information is shown in Table 35 below:

**Table 35. Dosing and Grouping**

| Grou p | Dosing group | N | Dosage | Dosing regimen | Dosing method |
|---|---|---|---|---|---|
| 1 | Blank control group | 3 | -- | QD | PO |
| 2 | Enhertu | 3 | 3 | Single dose (D9) | IV |
| 3 | IN10018 | 3 | 12.5 | QD*25 | PO |
| 4 | Enhertu +IN10018 | 3 | 3+12.5 | Single dose (D9) + QD*25 | IV+PO |

Note: N: number of animals; Dosing volume: adjusted according to the body weight of tumor-bearing mice (0.2 ml/20 g).

The main purpose is to detect the growth inhibitory effect or complete cure ability of the test drug on the human rectal cancer in vivo transplant tumor model LD1-0038-361855.

Measurement of tumor volume and body weight of tumor-bearing mice: Tumor volume was measured twice a week using a vernier caliper, and the formula for calculating tumor volume was $V = 0.5\, a \times b^2$, where a and b represent the long and wide diameters of the tumor, respectively;

Relative tumor proliferation rate T/C (%): The calculation formula is as follows:

$T/C\ \% = T_{RTV} / C_{RTV} \times 100\ \%$ ($T_{RTV}$: RTV of the treatment group; $C_{RTV}$: RTV of the negative control group). The relative tumor volume (RTV) was calculated based on the results of tumor measurement using the formula $RTV = T_{Vi} / T_{V0}$, where $T_{V0}$ is the average tumor volume measured at the time of grouping and dosing (i.e., $d_0$), and $T_{Vi}$ is the average tumor volume at a particular measurement. $T_{RTV}$ and $C_{RTV}$ data were taken on the same day.

Tumor growth inhibition rate TGI (%) = $[1-(T_{ti}-T_{t0})/ (V_{ci}-V_{c0})] \times 100$, $T_{ti}$ is the average tumor volume of the compound group after the start of dosing, $T_{t0}$ is the average tumor volume of the compound group at the first dose, $V_{c0}$ is the average tumor volume of the vehicle control group at the first dose, and $V_{ci}$ is the average tumor volume of the vehicle control group after the start of dose.

The body weight of all tumor-bearing mice was measured twice a week. The relative body weight change ratio after dosing was calculated: $RCBW(\%) = (BW_i - BW_0)/BW_0 \times 100$, where $BW_i$ is the body weight after the start of dosing and $BW_0$ is the body weight at the time of the first dose.

At the end of the experiment, the tumor masses were weighed and photographed.

**Data Analysis**

All data are expressed as mean ± SEM, where SEM = SD/SQRT (n), and n = number of animals in each experimental group. One-way ANOVA was used to compare tumor volumes between the treatment and control groups. All data were analyzed using GraphPad, and $p < 0.05$ was considered significant.

**Experimental results**

The results of tumor volume changes in each treatment group are shown in FIG. 20 and Table 36.

On the Day 24 after the start of dosing, the average tumor volume of tumor-bearing mice in the control group was 1379.41 ± 179.25 $mm^3$, and the average tumor volumes of tumor-bearing mice in the test drug Enhertu group, IN10018 group, and Enhertu + IN10018 group were 361.30 ± 113.01 $mm^3$, 1190.95 ± 463.62 $mm^3$, and 151.79 ± 85.19 $mm^3$, respectively; the tumor volume growth inhibition rate TGI (%) was 83.27%, 15.52%, and 100.39%, respectively; the relative tumor proliferation rate T/C (%) was 26.11%, 85.55%, and 10.98%, respectively. The experimental results showed that under the experimental conditions, the test drug Enhertu group, IN10018 group, and Enhertu+IN10018 group all showed statistically significant inhibitory effects on tumor growth in the in vivo rectal cancer transplant tumor model LD1-0038-361855.

At the end of the experiment (Day 24), all tumor-bearing mice were euthanized, and the subcutaneous tumors were excised and weighed. The average tumor weights for the control group, the test drug Enhertu group, the IN10018 group, and the Enhertu + IN10018 group were 1.420 ± 0.164 g, 0.278 ± 0.083 g, 1.078 ± 0.439 g, and 0.101 ± 0.058 g, respectively. Tumor weight results were generally consistent with tumor volume results.

**Table 37: Data Statistics**

| Group | Treatment group | *p value* VS vehicle | *p value* VS IN10018 | *p value* VS Enhertu |
|---|---|---|---|---|
| 1 | Blank control group | -- | -- | -- |
| 2 | Enhertu | 0.0382 | -- | -- |
| 3 | IN10018 | 0.9675 | -- | -- |
| 4 | Enhertu +IN10018 | 0.0125 | 0.0922 | 0.2129 |

**Example 10: In vivo efficacy evaluation of Enhertu and IN10018 as single drug or in combination in the human ovarian cancer xenograft model LD2-0032-200651**

Experimental animals: NU/NU mice, female, weighing 18-21 g, were purchased from Beijing Weitonglihua Laboratory Animal Technology Co., Ltd. and housed in the same environment as in Example 6.

Human ovarian cancer (poorly differentiated adenocarcinoma) tumor tissue (model number LD2-0032-200651) was passaged to FP3+5 and used in this efficacy experiment.

The test sample information is shown in Table 26.

The tumor masses of human ovarian cancer LD2-0032-200651 in vivo transplanted tumors were cut into pieces of approximately 3 mm × 3 mm × 3 mm (approximately 45 to 60 mg) and inoculated subcutaneously into NU/NU mice.

The mice were observed after inoculation and the growth of the tumor was monitored. On the Day 18 after inoculation, when the average tumor volume of the tumor-bearing mice reached 180.47 $mm^3$, the grouping and dosing were performed. The day of grouping and dosing was defined as Day 0. The specific grouping information is shown in Table 38 below:

**Table 38. Dosing and Grouping**

| Group | Dosing group | N | Dosage | Dosing regimen | Dosing method |
|---|---|---|---|---|---|
| 1 | Control group | 4 | -- | QD | PO |
| 2 | Enhertu | 4 | 3 | QW*3 | IV |
| 3 | IN10018 | 4 | 25 | QD | PO |
| 6 | Enhertu+IN10018 | 4 | 3+25 | QW*3+QD | IV+PO |

Note: N: number of animals; Dosing volume: adjusted according to the weight of tumor-bearing mice (0.2 ml/20 g);

The first dose of Enhertu began on the third day after grouping.

The main purpose is to detect the growth inhibitory effect or complete cure ability of the test drugs Enhertu and

IN10018 as single drug or in combination on the human ovarian cancer in vivo transplant tumor model LD2-0032-200651.

Tumor volume and tumor-bearing mouse weight were measured twice a week using a vernier caliper. The tumor volume was calculated as $V = 0.5\ a \times b^2$, where a and b represent the long and wide diameters of the tumor, respectively; Relative tumor proliferation rate T/C (%): The calculation formula is as follows:

$$T/C\ \% = T_{RTV} / C_{RTV} \times 100\ \%$$

($T_{RTV}$: RTV of the treatment group; $C_{RTV}$: RTV of the negative control group).

The relative tumor volume (RTV) was calculated based on the results of tumor measurement using the formula $RTV = T_{Vi}/T_{V0}$, where $T_{V0}$ is the average tumor volume measured at the time of grouping and dosing (i.e., do), and $T_{Vi}$ is the average tumor volume at a particular measurement. $T_{RTV}$ and $C_{RTV}$ data were taken on the same day.

Tumor growth inhibition rate TGI (%) = $[1-(T_{ti}-T_{t0})/ (V_{ci}-V_{c0})] \times 100$, $T_{ti}$ is the average tumor volume of the compound group after the start of dosing, $T_{t0}$ is the average tumor volume of the compound group at the first dose, $V_{c0}$ is the average tumor volume of the vehicle control group at the first dose, and $V_{ci}$ is the average tumor volume of the vehicle control group after the start of dose.

The body weight of all tumor-bearing mice was measured twice weekly. The relative weight change after dosing was calculated: $RCBW(\%) = (BW_i - BW_0)/BW_0 \times 100$, where $BW_i$ is the weight after the start of dosing and $BW_0$ is the weight at the time of the first dose. Tumor masses were weighed and photographed at the end of the experiment.

**Data Analysis**

All data are expressed as mean ± SEM, where SEM = SD/SQRT (n), and n = number of animals in each experimental group. One-way ANOVA was used to compare tumor volumes between the treatment and control groups. All data were analyzed using GraphPad, and $p < 0.05$ was considered significant.

**Experimental results**

The results of tumor volume changes in each treatment group are shown in FIG. 21 and Table 39.

Table 39:

| Group | Treatmen t group | Tumor volume /Day 0 | Tumor volume /Day 21 | *p value* VS vehicle | *p value* VS G4 | Relative tumor volume / Day 21 | T/C(%)/ Day 21 | TGI(%) /Day 21 | Tumor weight (g) / Day 21 |
|---|---|---|---|---|---|---|---|---|---|
| 1 | Control group | 179.51± 31.37 | 2132.47 ±513.67 | -- | -- | 9.07 | -- | -- | 2.075±0. 466 |
| 2 | Enhertu | 181.25± 16.18 | 1294.64 ±285.36 | 0.1816 | 0.1067 | 5.54 | 61.08 | 43.20 | 1.212±0. 271 |
| 3 | IN10018 | 179.67± 22.16 | 1289.97 ±128.06 | 0.1785 | 0.0177 | 5.26 | 58.04 | 47.11 | 1.189±0. 139 |
| 4 | Enhertu+ IN10018 | 181.46± 28.81 | 700.63± 129.27 | 0.0167 | -- | 2.92 | 32.15 | 76.00 | 0.711±0. 137 |

On Day 21 after the start of dosing, the average tumor volume of tumor-bearing mice in the control group was 2132.47 ± 513.67 $mm^3$. The average tumor volumes of tumor-bearing mice in the Enhertu 3 mg/kg QW*3 group, the IN10018 25 mg/kg QD*21 group, and the IN10018 25 mg/kg QD*21 combined with Enhertu 3 mg/kg QW*3 group were 1294.64 ± 285.36 $mm^3$, 1289.97 ± 128.06 $mm^3$, and 700.63 ± 129.27 $mm^3$, respectively. The tumor volume growth inhibition rates TGI (%) were 43.20%, 47.11% and 76.00% respectively; the relative tumor proliferation rates T/C (%) were 61.08%, 58.04% and 32.15% respectively. The experimental results showed that under the experimental conditions of this study, the combination of the test drug IN10018 25 mg/kg QD*21 and Enhertu 3 mg/kg QW*3 showed a statistically significant inhibitory effect on tumor growth in the ovarian cancer in vivo transplant tumor model LD2-0032-200651. The tumor volume in the IN10018 25 mg/kg QD*21 combined with Enhertu 3 mg/kg QW*3 group was smaller than that in each single-drug group, but there was no statistically significant difference compared with the Enhertu 3 mg/kg QW*3 single-drug group.

On the final day of the experiment (Day 21), all tumor-bearing mice were euthanized, and the subcutaneous

tumors were excised and weighed. The average tumor weights for the control group, the Enhertu 3 mg/kg QW*3 group, the IN10018 25 mg/kg QD*21 group, and the IN10018 25 mg/kg QD*21 combined with Enhertu 3 mg/kg QW*3 group were 2.075 ± 0.466 g, 1.212 ± 0.271 g, 1.189 ± 0.139 g, and 0.711 ± 0.137 g, respectively. Tumor weight results were generally consistent with tumor volume results.

The body weight of experimental animals can be used as an indirect indicator for monitoring drug toxicity. No mice in any of the drug-dosing groups showed significant weight loss after dosing, indicating that tumor-bearing mice tolerated the drugs well at the experimental doses.

**Example 11: Evaluation of the anti-tumor effects of Enhertu and IN10018 in female NOD-SCID mice in the LU-01-1626 human lung cancer patient-derived xenograft (PDX) model**

Experimental animals: NOD SCID mice, female, weighing 18-20 g, purchased from Saiye Biotechnology Co., Ltd., and housed in the same environment as in Example 6.

Human lung cancer tumor tissue, passaged to FP5, was used in this efficacy experiment.

The test sample information is shown in Table 26.

Each mouse was inoculated subcutaneously with a LU-01-1626 FP5 tumor piece (approximately 30 $mm^3$) on the right flank for tumor growth. Treatment began on Day 18 after tumor implantation, when the average tumor size reached approximately 109 $mm^3$. The animals were divided into groups using Excel-based randomization software and stratified randomization was performed according to their tumor volume. Each group consisted of 4 tumor-bearing mice. The test samples were administered to the mice according to the protocol shown in Table 40 of the experimental design.

Table 40:

| Group | $N^1$ | Dosing group | Dosage (mg/kg) | Dosing volume ($\mu L/g$)$^2$ | Dosing method | Dosing regimen |
|---|---|---|---|---|---|---|
| 1 | 4 | Control group | -- | 10 | PO | QDx32 |
| 2 | 4 | Enhertu | 10/3/6 | 10 | IV | QWx6 doses |
| 3 | 4 | IN10018 | 25/12.5 | 10 | PO | QDx32 |
| 4 | 4 | Enhertu+IN10018 | 10/3/6+25/12.5 | 10+10 | IV+PO | QWx6 doses+QDx39 |
| Note: 1. N: animal number;<br>2. Dosing volume: The dosage volume was adjusted based on 10 μL/g body weight. | | | | | | |

The primary endpoint was whether tumor growth could be delayed or if the mice could be cured. Tumor volume and tumor-bearing mouse weight were measured twice a week using a vernier caliper. The tumor volume was calculated as $V = 0.5\ a \times b^2$, where a and b represent the long and wide diameters of the tumor, respectively.

Tumor growth inhibition rate TGI (%) = $[1-(T_{ti}-T_{t0})/ (V_{ci}-V_{c0})] \times 100$, $T_{ti}$ is the average tumor volume of the compound group after the start of dosing, $T_{t0}$ is the average tumor volume of the compound group at the first dose, $V_{c0}$ is the average tumor volume of the vehicle control group at the first dose, and $V_{ci}$ is the average tumor volume of the vehicle control group after the start of dose.

Relative tumor proliferation rate T/C (%): The calculation formula is as follows:

$$T/C\ \% = T_{RTV} / C_{RTV} \times 100\ \%$$

($T_{RTV}$: RTV of the treatment group; $C_{RTV}$: RTV of the negative control group).

The relative tumor volume (RTV) was calculated based on the results of tumor measurement using the formula $RTV = T_{Vi}/T_{V0}$, where $T_{V0}$ is the average tumor volume measured at the time of grouping and dosing (i.e., do), and $T_{Vi}$ is the average tumor volume at a particular measurement. $T_{RTV}$ and $C_{RTV}$ data were taken on the same day.

The body weight of all tumor-bearing mice was measured twice a week. The relative weight change ratio after dosing was calculated: RCBW(%) = $(BW_i - BW_0)/BW_0 \times 100$, where $BW_i$ is the weight after the start of dosing and $BW_0$ is the weight at the time of the first dose.

**Data Analysis**

Summary statistics of tumor volume at each time point for each group are provided, including the mean and

standard error of the mean (SEM).

The data obtained on Day 31 after the start of treatment were statistically analyzed for differences in tumor volume among the groups.

One-way analysis of variance was performed to compare tumor volumes between groups. Since no significant F statistic (ratio of treatment variance to error variance) was obtained, the Games-Howell test was used for comparisons between groups. All data were analyzed using SPSS 29.0, and $p < 0.05$ was considered statistically significant.

The results of tumor volume changes in each treatment group are shown in FIG. 22 and Table 41.

Table 41:

| **Dosing group** | **Tumor volume ($mm^3$)[1] Day 31** | **T/C[2] (%)** | **TGI[3] (%)** | ***P value[4]*** |
|---|---|---|---|---|
| Control group | 1,977 ± 135 | -- | -- | -- |
| Enhertu, 10/3/6 mg/kg | 664 ± 157 | 33.58 | 70.28 | 0.006 |
| IN10018, 25/12.5 mg/kg | 1,459 ± 123 | 73.80 | 27.74 | 0.177 |
| Enhertu, 10/3/6 mg/kg + IN10018, 25/12.5 mg/kg | 405 ± 73 | 20.49 | 84.16 | 0.002 |

Note: 1. Mean ± SEM;
2. Tumor growth inhibition was calculated by dividing the group mean tumor volume of the treatment group by the group mean tumor volume of the control group (T/C);
3.TGI(%)=[1-(T31-T0)/(V31-V0)]×100;
4. p-value was calculated based on tumor size.
On day 31 after the start of dosing, the average tumor volume of tumor-bearing mice in the control group was 1977 ± 135 $mm^3$, and the average tumor volumes of tumor-bearing mice in the Enhertu group, IN10018 group, and IN10018 combined with Enhertu group were 664 ± 157 $mm^3$, 1459 ± 123 $mm^3$, and 405 ± 73 $mm^3$, respectively;
The tumor volume growth inhibition rates TGI (%) were 70.28%, 27.74% and 84.16% respectively; the relative tumor proliferation rates T/C (%) were 33.58%, 73.80% and 20.49% respectively.

The experimental results showed that the tumor inhibition effect of the two-drug combination group was better than that of the single drug group.

**Example 12: Evaluation of the anti-tumor effects of test drugs Ab6000-DXD and IN10018** in **female BALB/c nude mice in the OVCAR-3 human ovarian cancer (CDX) model**

Experimental animals: BALB/c nude mice, female, 6-8 weeks old, purchased from Beijing Weitonglihua Experimental Animal Technology Co., Ltd., and housed in the same environment as in Example 6.

**OVCAR-3 human ovarian cancer cells were purchased from Nanjing Cobioer Biotechnology Co., Ltd.** To establish subcutaneous tumors, OVCAR-3 human ovarian cancer cells were harvested by trypsinization, centrifuged, washed, and suspended in ice-cold PBS + 5% FCS and growth factor-reduced Matrigel (1: 1) at a cell concentration of 1 x $10^7$ cells/ml. Then, 200 μl of the cell suspension containing OVCAR-3 cells was subcutaneously injected into the right flank of nude mice (one site per mouse). When tumor volume reached 217 $mm^3$ (day 31 after cell injection), mice were randomly assigned to treatment groups or blank control group. Group information is shown in Table 42.

Table 42:

| **Group** | **N[1]** | **Dosing group** | **dosage (mg/kg)** | **Dosing method** | **Dosing regimen** |
|---|---|---|---|---|---|
| 1 | 5 | Control group | -- | PO | QDx14 |
| 2 | 5 | Ab6000-DXD | 1/3 | IV | 1mg/kg(d(0))/ 3mg/kg(d7))QW |
| 3 | 5 | IN10018 | 12.5 | PO | QDx14 |
| 4 | 5 | Ab6000-DXD +IN 10018 | 1/3+12.5 | IV+PO | 1mg/kg(d(0))/ 3mg/kg(d7))QW+ QDx14 |

Tumor diameters were measured twice a week (Monday and Thursday) using a caliper. The volume of each tumor (in $mm^3$) was calculated according to the formula, "tumor volume = length x diameter$^2$ x 0.5". To monitor the side effects of treatment, mice were examined daily for abnormalities and their body weights were measured twice a week (Monday and Thursday). Animals were sacrificed at the end of the study; during the study, animals with tumor necrosis or

tumor size exceeding 2000 $mm^3$ were sacrificed early for ethical reasons.

IN 10018 information is shown in Table 26.

**The full-length sequence of Ab6000 is as follows:**

**Heavy chain sequence: VH-** CH (Human IgG1 Mutation):

EVQLVQSGAEVKKPGASVKVSCKASGYTFTRNFMHWVRQAPGQGLEWMGWIYPGDGETE YAQKFQGRVTITADTSTSTAYMELSSLRSEDTAVYYCARGVYGGFAGGYFDFWGQGTLVTVSSAST KGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPEAAGGPSVFLFPPKPKDTLMISRTP EVTCVVVDVSHEDPEVKFNWYVDGVEVHNAKTKPREEQYNSTYRVVSVLTVLHQDWLNGKEYKC KVSNKALAAPIEKTISKAKGQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQKSLSLSPGK

chain sequence: VL-CL(Human Kappa):

DIQMTQSPSSLSASVGDRVTITCKASQNIYKNLAWYQQKPGKAPKLLIYDANTLQTGVPSRF SGSGSGSDFTLTISSLQPEDFATYFCQQYYSGWAFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC

Preparation of conjugated drug Ab6000-DXD ADC

An appropriate amount of reducing agent (TCEP·HCl, Thermo, CAS No.: 51805-45-9) was added to reduce the antibody (Ab6000) to expose the sulfhydryl groups between the antibody chains. The small molecule Deruxtecan (MCE, CAS No.: 1599440-13-7) was dissolved in DMSO and prepared into a 10 mM solution.

The dissolved solution was added at a molar ratio of 15: 1 between the antibody and the linker-payload. The antibody and the linker+payload were coupled together through a Michael addition reaction between the thiol group and the maleimide group on the linker.

The mixture after coupling was subjected to buffer replacement to remove residual small molecules. The purity and DAR (drug/antibody ratio) value of the antibody conjugate after the reaction were determined by HPLC-SEC (size exclusion chromatography) and HPLC-HIC (hydrophobic chromatography), respectively. The coupling results are shown in Table 43.

Table 43: ADC drug conjugate preparation

| Sample name (ADC) | Purity | DAR |
|---|---|---|
| Ab6000-DXD | 97.7% | 7.87 |

The tumor volumes of each treatment group are shown in FIG. 23. On day 14 after dosing, the control group had a tumor volume of 616 $mm^3$, the ab6000-DXD singe drug group had a tumor volume of 425 $mm^3$, the IN10018 monotherapy group had a tumor volume of 510 $mm^3$, and the ab6000-DXD and IN10018 combination group had a tumor volume of 344 $mm^3$. These data demonstrate that the two-drug combination group exhibited superior tumor inhibition compared to the singe drug groups.

All references mentioned in this application are incorporated herein by reference in their entirety, just as if each reference were listed separately. It should be understood that after reading the disclosure of this application, those skilled in the art may make various changes or modifications to the present invention, and these equivalent forms also fall within the scope of the claims appended hereto.

## Claims

1. Use of IN10018 or a pharmaceutically acceptable salt thereof in the manufacture of a medicament for increasing the

concentration of a macromolecular drug in tumor tissue, wherein the macromolecular drug is a monoclonal antibody, a bispecific antibody, or an antibody conjugate, wherein the structure of IN10018 is as follows:

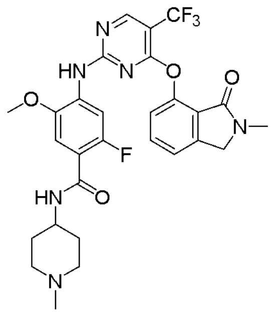

.

2. A pharmaceutical combination product comprising IN10018 or a pharmaceutically acceptable salt thereof and a macromolecular drug for use in treating a tumor in a subject, wherein the macromolecular drug is a monoclonal antibody, a bispecific antibody, or an antibody conjugate, wherein the structure of IN10018 is as follows:

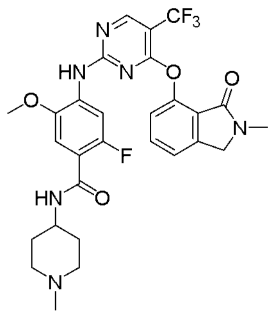

.

3. Use of IN10018 or a pharmaceutically acceptable salt thereof for increasing the concentration of a macromolecular drug in tumor tissue, wherein the macromolecular drug is a monoclonal antibody, a bispecific antibody, or an antibody conjugate, wherein the structure of IN10018 is as follows:

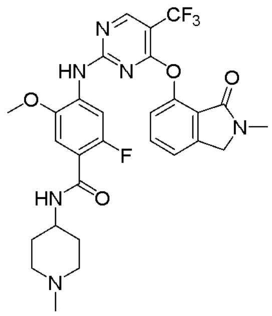

.

4. A method for treating tumors, comprising administering to a subject in need thereof a therapeutically effective amount of IN10018 or a pharmaceutically acceptable salt thereof and a macromolecular drug, wherein the macromolecular drug is a monoclonal antibody, a bispecific antibody, or an antibody conjugate, wherein the structure of IN10018 is as follows:

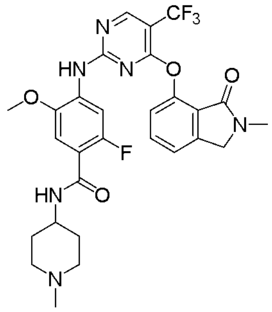

.

**5.** A kit or a pharmaceutically acceptable composition comprising:

(a) IN10018 or a pharmaceutically acceptable salt thereof; and
(b) a macromolecular drug, wherein the macromolecular drug is a monoclonal antibody, a bispecific antibody, or an antibody conjugate;

the structure of IN10018 is as follows:

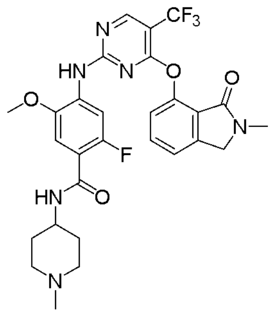

.

**6.** The use, pharmaceutical combination product, method, kit or pharmaceutically acceptable composition according to any one of claims 1 to 5, wherein the pharmaceutically acceptable salt of IN10018 is a tartrate salt.

**7.** The use, pharmaceutical combination product, method, kit or pharmaceutically acceptable composition according to any one of claims 1 to 6, wherein the monoclonal antibody is Racotumomab, Rituximab, 131I-Metuximab, JMT-103, Necitumumab, Alemtuzumab, Elotuzumab, Bevacizumab, Ofatumumab, Tocilizumab, Atezolizumab, Toripalimab, HX-008, Camrelizumab, Ocrelizumab, Sugemalimab, Lenzilumab, Sintilimab, Vilobelimab, Margetuximab, Siltuximab, Mogamulizumab, amivantamab, Cadonilimab, Inebilizumab, iodine I 131 derlotuximab biotin, Isatuximab, Serplulimab, Retifanlimab, Cetuximab, Adebrelimab, Tislelizumab, Penpulimab, Teprotumumab, Itolizumab, Dostarlimab, Denosumab, Obinutuzumab, Nimotuzumab, Teclistamab, Daratumumab, dinutuximab, Tafasitamab, Socazolimab, Dupilumab, Prolgolimab, Blinatumomab, Geptanolimab, Panitumumab, Canakinumab, Ramucirumab, envafolimab, Belimumab, Leronlimab, Ranibizumab, Natalizumab, Cosibelimab, Zimberelimab, Trastuzumab, Catumaxomab, Durvalumab, avelumab, Ublituximab, Cemiplimab, Pembrolizumab, Glofitamab, Pertuzumab, Bermekimab, tremelimumab, ipilimumab, Naxitamab, infliximab, nivolumab, Omburtamab, Crizanlizumab, burosumab, Talquetamab, Besilesomab, Alirocumab, Arcitumomab, or a biosimilar thereof; particularly, Trastuzumab or a biosimilar thereof; preferably, Trastuzumab.

**8.** The use, pharmaceutical combination product, method, kit or pharmaceutically acceptable composition according to any one of claims 1 to 6, wherein the bispecific antibody is Teclistamab, Blinatumomab, Cadonilimab, Mosunetuzumab, Catumaxomab, Ublituximab, amivantamab, Talquetamab, Epcoritamab, Glofitamab, Zanidatamab, Elranatamab, Tebotelimab, amivantamab, SI-B001, Odronextamab, KN-026, KN-046, Ivonescimab, SHR-1701, M7824, GEN-3009, Navicixizumab, GB-261, CM-355, Plamotamab, or a biosimilar thereof.

**9.** The use, pharmaceutical combination product, method, kit or pharmaceutically acceptable composition according to any one of claims 1 to 6, wherein the antibody conjugate is Loncastuximab Tesirine, Ibritumomab Tiuxetan, Tisotumab Vedotin, Sacituzumab Govitecan, Enfortumab Vedotin, Inotuzumab Ozogamicin, Gemtuzumab Ozogamicin, Belantamab Mafodotin, Trastuzumab Emtansine, Moxetumomab Pasudotox, Polatuzumab Vedotin, Disitamab Vedotin,

Brentuximab Vedotin, Trastuzumab deruxtecan(DS-8201), Trastuzumab Emtansine, Cetuximab Sarotalocan, Mirvetuximab Soravtansine, Trastuzumab Duocarmazine, ARX-788, KL-A264, Upifitamab Rilsodotin, Tusamitamab Ravtansine, Naptumomab Estafenatox, Datopotamab Deruxtecan, Oportuzumab Monatox, SHR-A1811, Patritumab Deruxtecan, Telisotuzumab vedotin, Trastuzumab Duocarmazine, MK-2140, TAA-013, BIO-106, DB-1305, MRG-004A, AZD-8205, ADCT-602, FOR-46, TPX-4589, BMS-986148, SOT-102, ESG-401, CD117-ADC, LCB-14, W-0101, REGN-5093-M114, CX-2029, BDC-1001, DGN549-C (Pivekimab Sunirine), AVID-100, MRG-003, CAT-5001, HDP-101, MORAb-202 (Farletuzumab Ecteribulin), L-DOS47, Praluzatamab Ravtansine, BA3021(Ozuriftamab Vedotin), DS-7300, BA3011(Mecbotamab Vedotin), Camidanlumab Tesirine, Ladiratuzumab Vedotin, LMB-2, SAT-012, DEBIO-1562, PSMA-ADC, MRG-002, OBI-999, Coltuximab Ravtansine, KL-A166, DX-126-262, NBE-002, OXS-1550, Lorvotuzumab Mertansine, TAC-001, MT-8633, TRPH-222, TAK-164, STRO-001, CX-2043, TBL-0306M, HDP-103, DAN-311, PRO-1102, GM-103 ADC, TE-1218, TE-1112, T-PNU, BV-001, DS-1062, SKB264, Ab6000-Dxd, or a biosimilar thereof; particularly, ESG-401, DS-1062, SKB264, Sacituzumab Govitecan, Ab6000-Dxd, or a biosimilar thereof; preferably, ESG-401, DS-1062, SKB264, Sacituzumab Govitecan/Trastuzumab deruxtecan (DS-8201) or Ab6000-Dxd.

**10.** The use, pharmaceutical combination product, method, kit or pharmaceutically acceptable composition according to any one of claims 1 to 6, wherein the macromolecular drug is an antibody conjugate, in particular an antibody conjugate of Trop-2, preferably ESG-401, DS-1062, SKB264 or Sacituzumab Govitecan.

**11.** The use, pharmaceutical combination product, method, kit or pharmaceutically acceptable composition according to any one of claims 1 to 10, wherein the IN10018 or a pharmaceutically acceptable salt thereof and the macromolecular drug are administered to the subject simultaneously or sequentially.

**12.** The use, pharmaceutical combination product, method, kit or pharmaceutically acceptable composition according to any one of claims 1 to 11, for treating tumors.

**13.** The use, pharmaceutical combination product, method, kit or pharmaceutically acceptable composition according to claim 12, wherein the tumor is selected from bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastic tumor, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, bile duct cancer, leiomyosarcoma, liposarcoma, nasopharyngeal cancer, neuroendocrine cancer, ovarian cancer, salivary gland cancer, metastatic tumor caused by spindle cell carcinoma, anaplastic large cell lymphoma, undifferentiated thyroid cancer, non-Hodgkin's lymphoma, Hodgkin's lymphoma, glioma and hematological malignancies, such as acute myeloid leukemia (AML), acute lymphocytic leukemia (ALL), diffuse large B-cell lymphoma (DLBCL), follicular lymphoma (FL), chronic lymphocytic leukemia (CLL), chronic granulocytic leukemia (CML); preferably, the tumor is gastric cancer, lung cancer, breast cancer, glioma, esophageal cancer, pancreatic cancer, head and neck cancer, colon cancer or ovarian cancer; more preferably, the tumor is gastric cancer, breast cancer, pancreatic cancer, colon cancer, ovarian cancer or lung cancer.

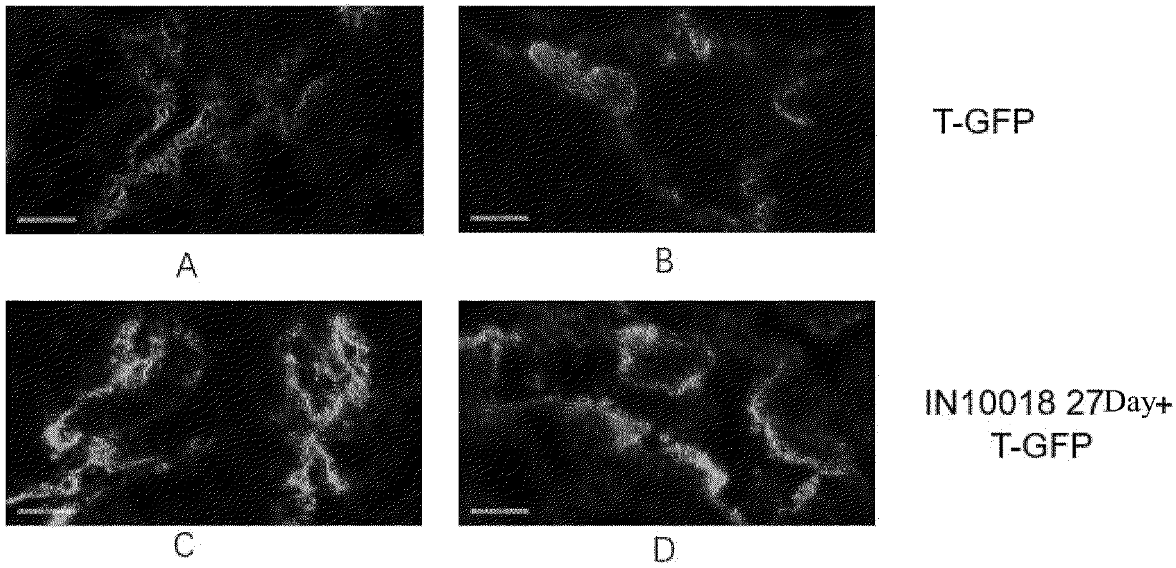
T-GFP
A
B
C
D
IN10018 27Day+
T-GFP

FIG. 1

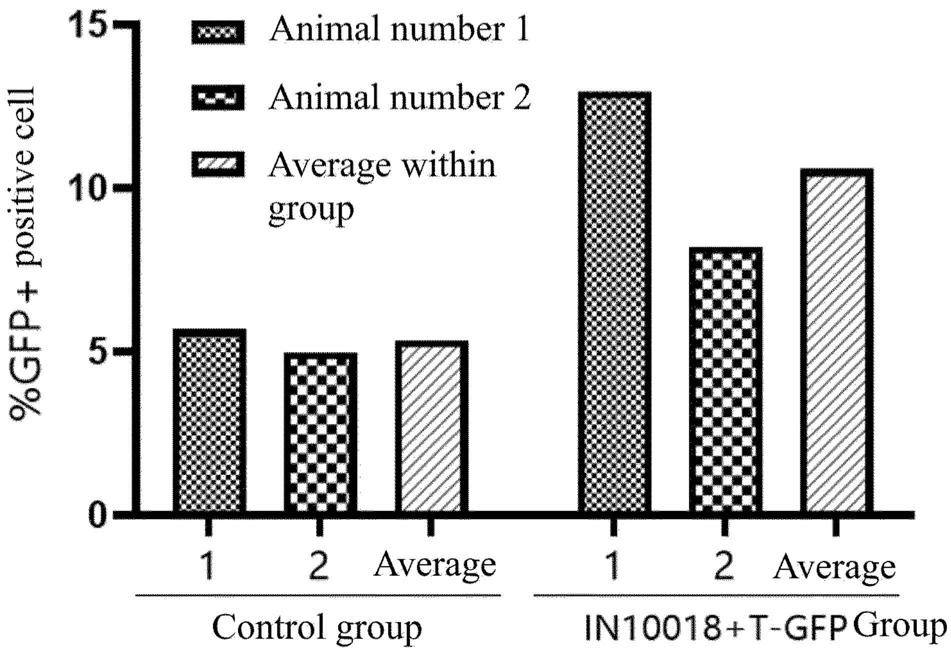
15
10
5
0
%GFP + positive cell
Animal number 1
Animal number 2
Average within group
1
2
Average
1
2
Average
Control group
IN10018+T-GFP Group

FIG. 2

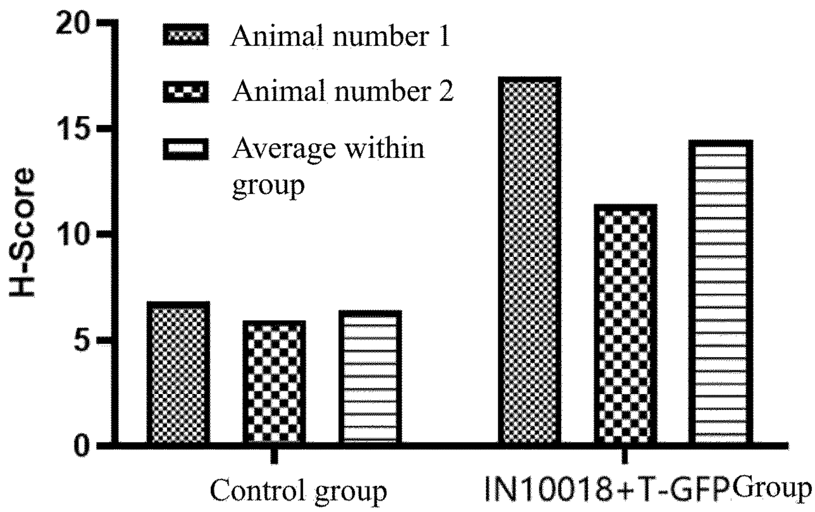


FIG. 3

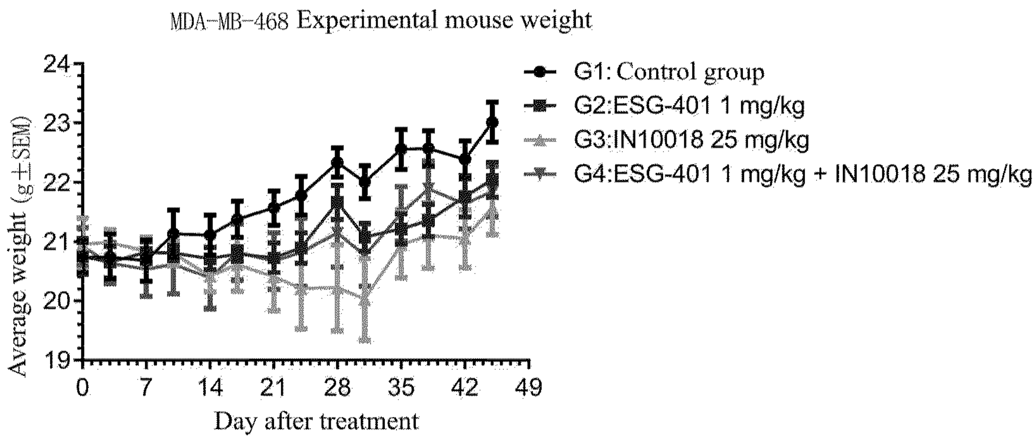


FIG. 4

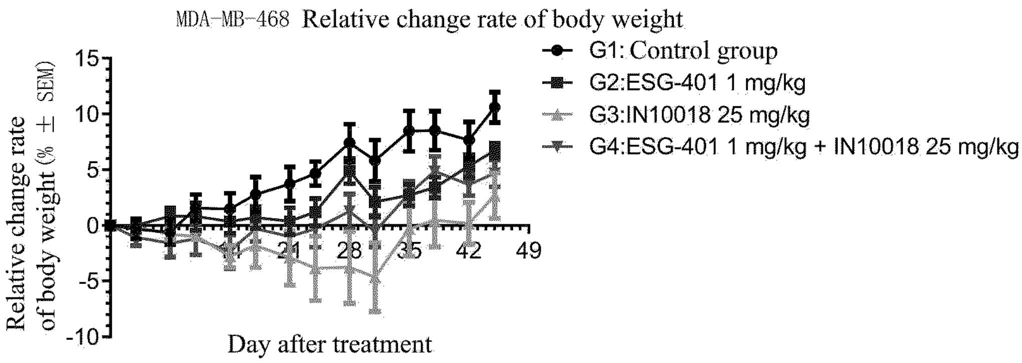


FIG. 5

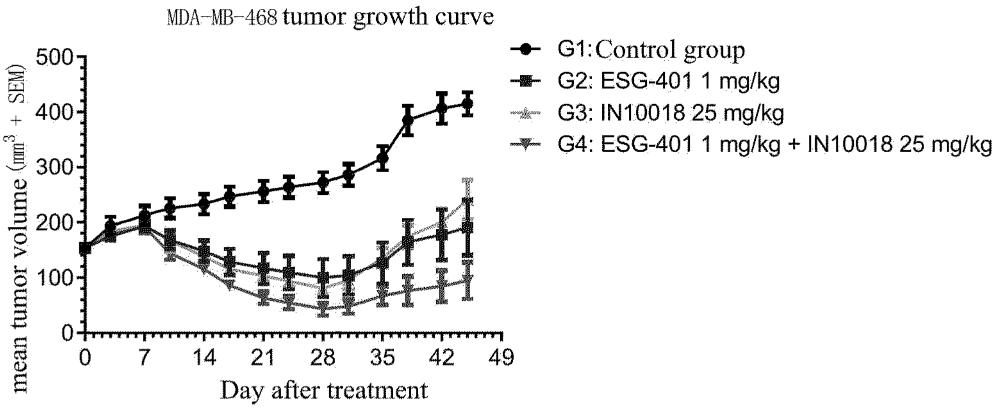
MDA-MB-468 tumor growth curve
mean tumor volume (mm³ + SEM)
500
400
300
200
100
0
0
7
14
21
28
35
42
49
Day after treatment
G1: Control group
G2: ESG-401 1 mg/kg
G3: IN10018 25 mg/kg
G4: ESG-401 1 mg/kg + IN10018 25 mg/kg

FIG. 6

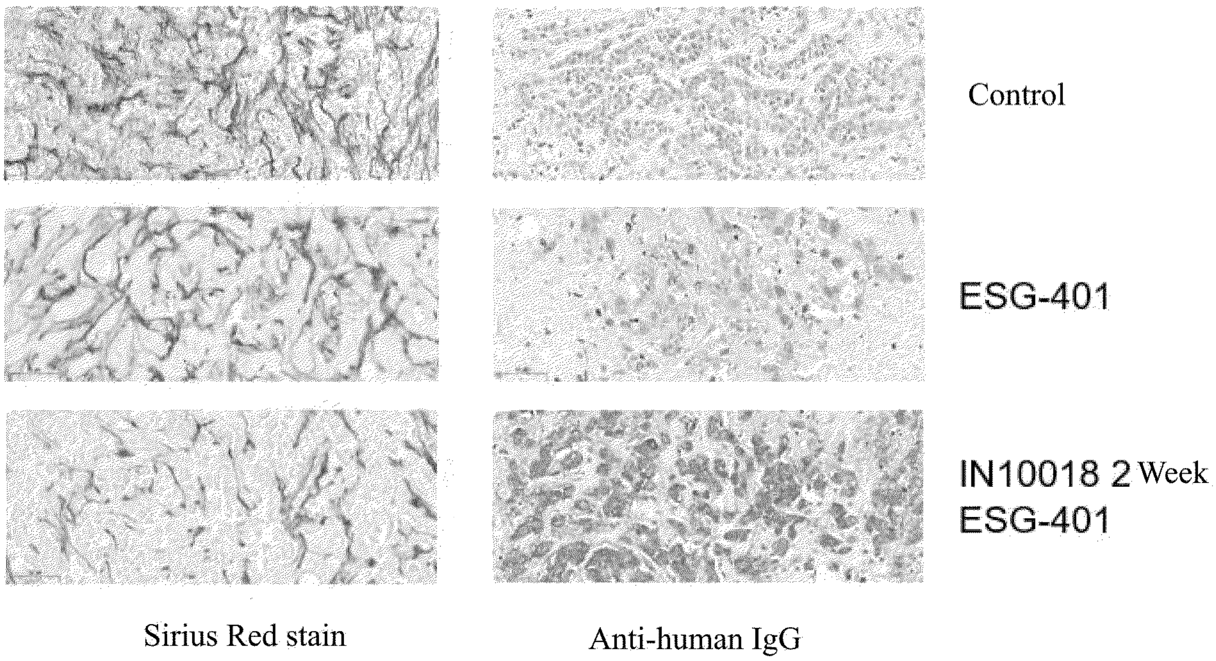
Control
ESG-401
IN10018 2 Week
ESG-401
Sirius Red stain
Anti-human IgG

FIG. 7

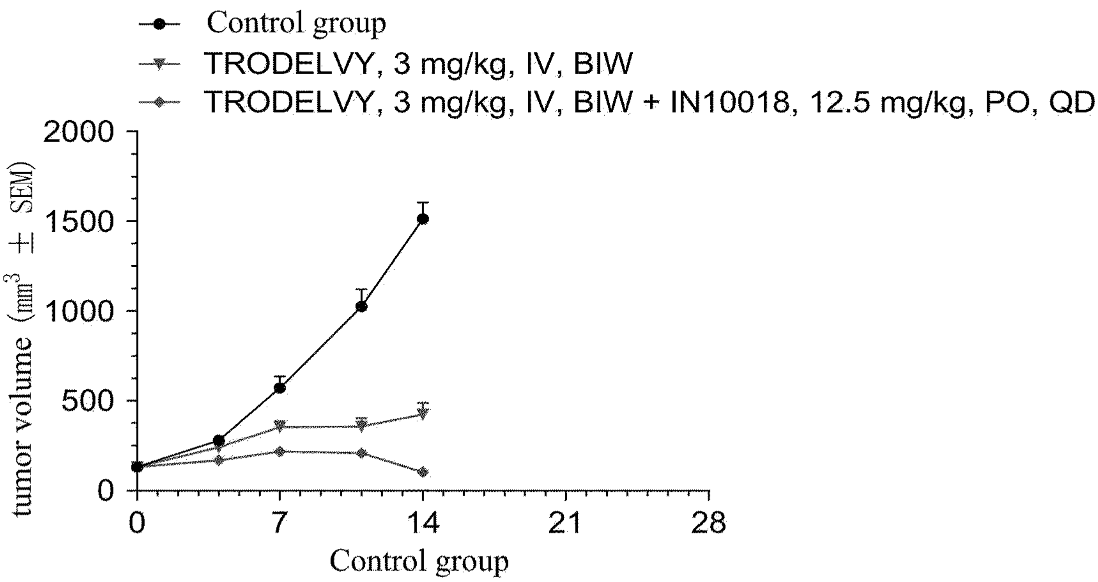
Control group
TRODELVY, 3 mg/kg, IV, BIW
TRODELVY, 3 mg/kg, IV, BIW + IN10018, 12.5 mg/kg, PO, QD
2000
1500
1000
500
0
tumor volume (mm3 ± SEM)
0
7
14
21
28
Control group

FIG. 8

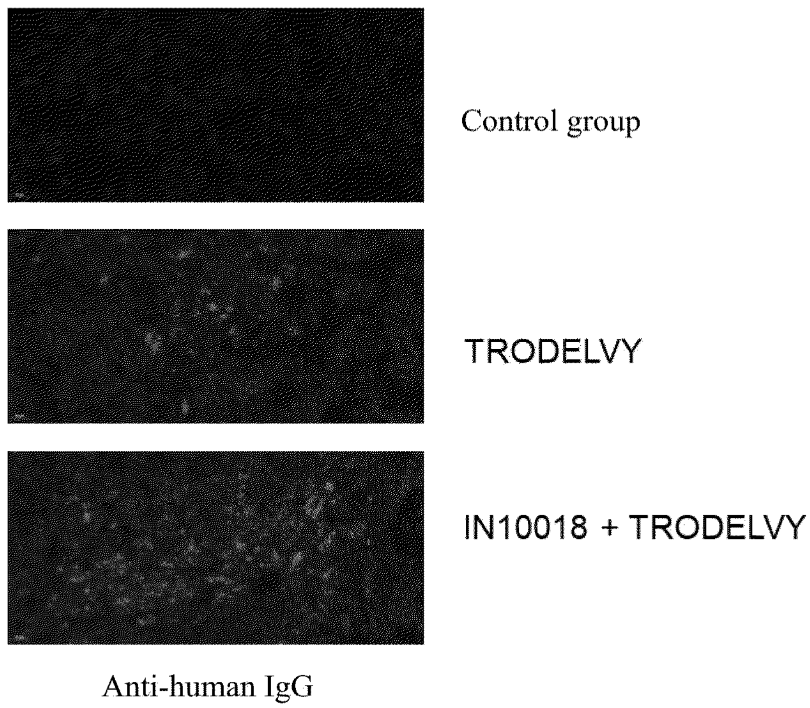
Control group
TRODELVY
IN10018 + TRODELVY
Anti-human IgG

FIG. 9

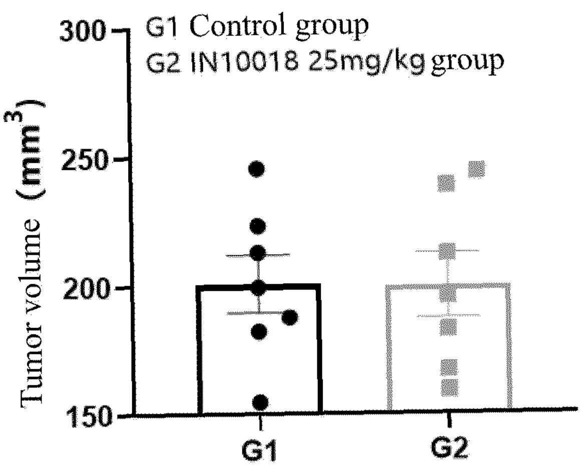
G1 Control group
G2 IN10018 25mg/kg group
Tumor volume (mm$^3$)
300
250
200
150
G1
G2

FIG. 10

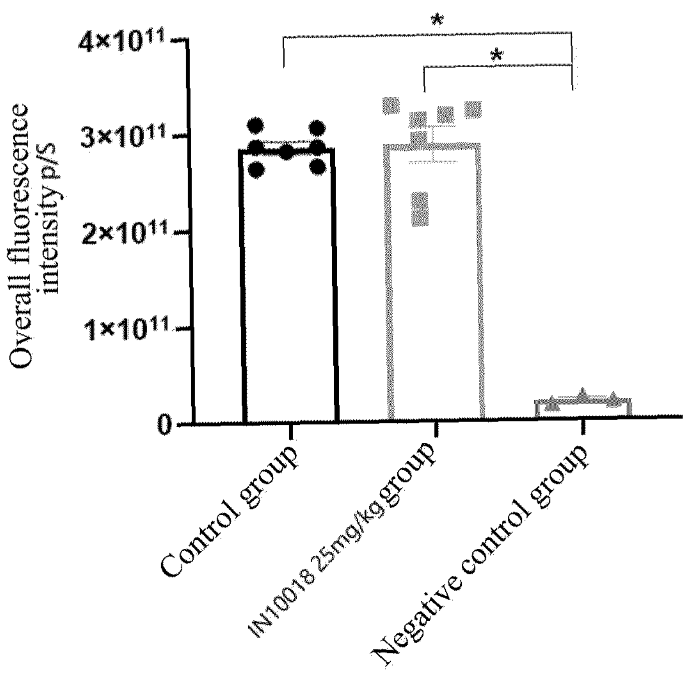
*
*
Overall fluorescence intensity p/S
4×10$^{11}$
3×10$^{11}$
2×10$^{11}$
1×10$^{11}$
0
Control group
IN10018 25mg/kg group
Negative control group

FIG. 11

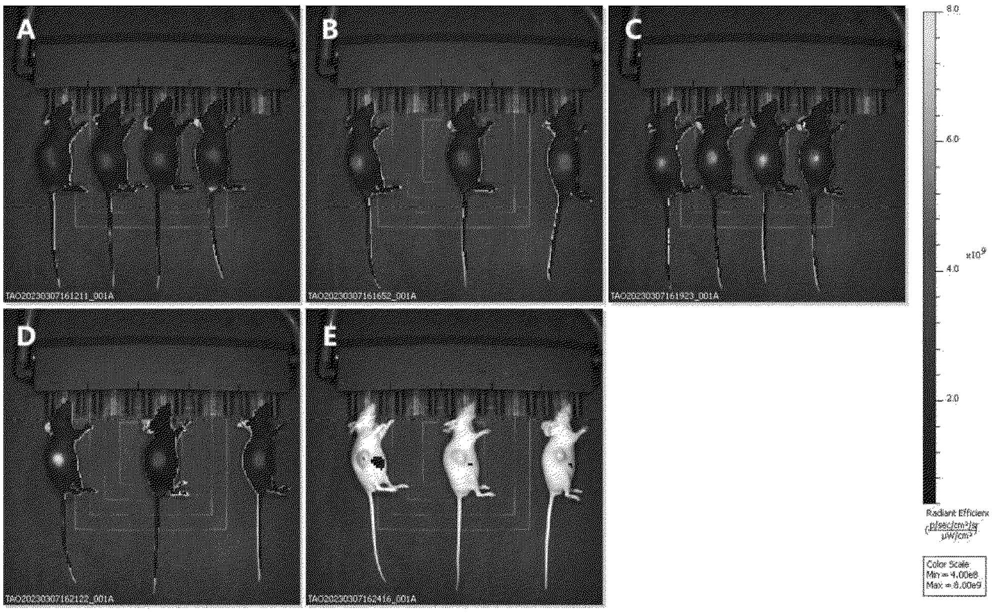
A
B
C
D
E
Radiant Efficiency
Color Scale
Min = 4.00e8
Max = 8.00e9

FIG. 12

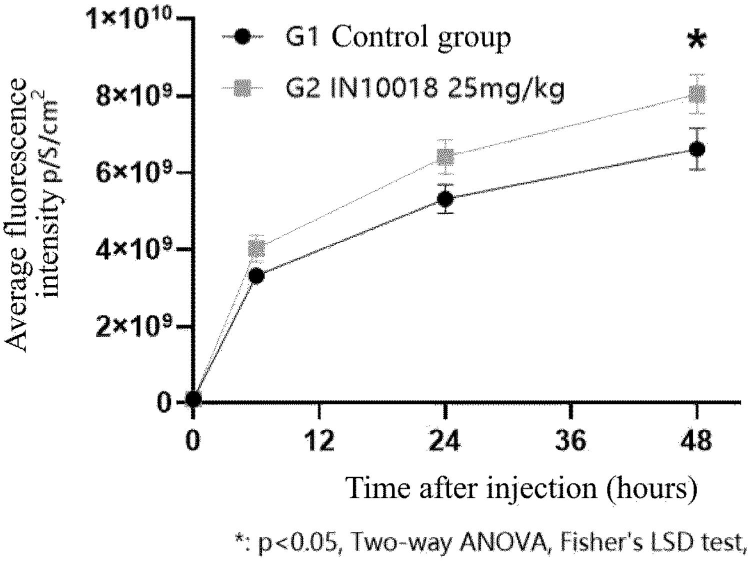
G1 Control group
G2 IN10018 25mg/kg
Average fluorescence intensity p/S/cm2
Time after injection (hours)
*: p<0.05, Two-way ANOVA, Fisher's LSD test,

FIG. 13

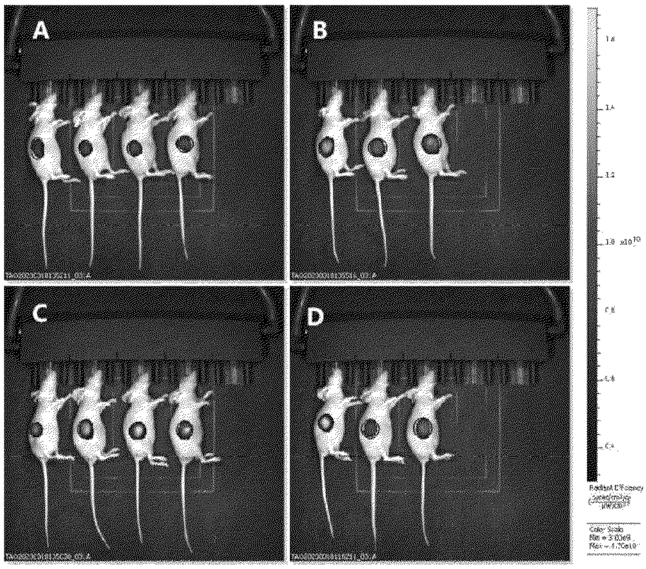
A
B
C
D

FIG. 14

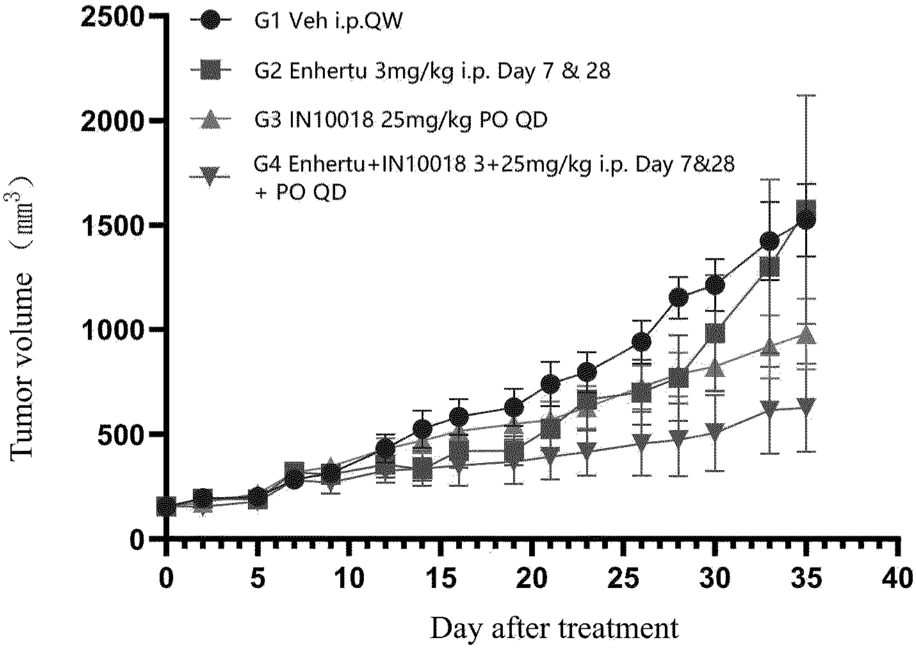
G1 Veh i.p.QW
G2 Enhertu 3mg/kg i.p. Day 7 & 28
G3 IN10018 25mg/kg PO QD
G4 Enhertu+IN10018 3+25mg/kg i.p. Day 7&28 + PO QD
Tumor volume （㎜³）
2500
2000
1500
1000
500
0
0
5
10
15
20
25
30
35
40
Day after treatment

FIG. 15

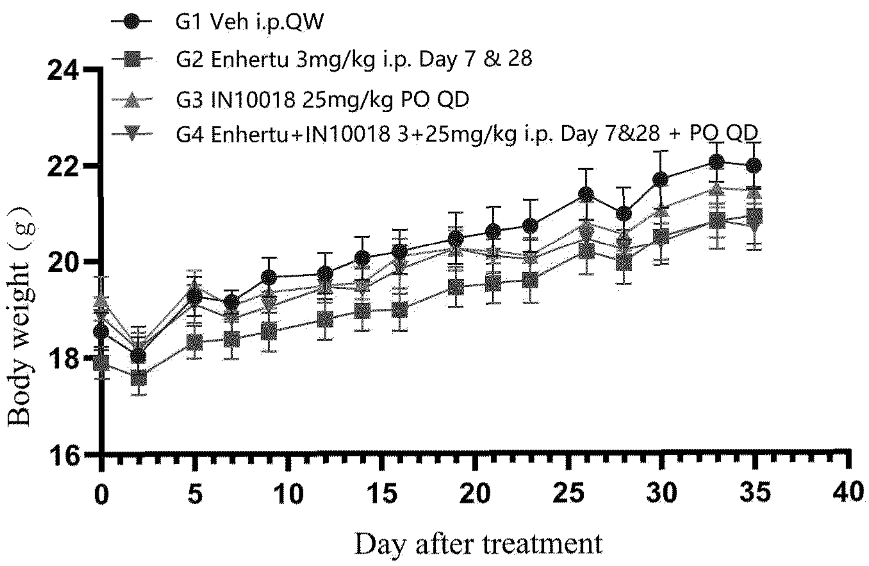
G1 Veh i.p.QW
G2 Enhertu 3mg/kg i.p. Day 7 & 28
G3 IN10018 25mg/kg PO QD
G4 Enhertu+IN10018 3+25mg/kg i.p. Day 7&28 + PO QD
Body weight（g）
Day after treatment
16
18
20
22
24
0
5
10
15
20
25
30
35
40

FIG. 16

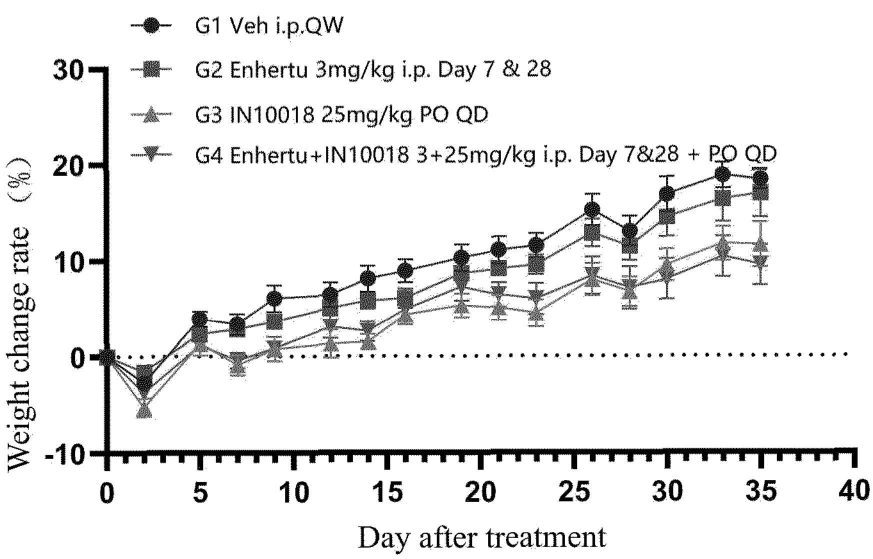
G1 Veh i.p.QW
G2 Enhertu 3mg/kg i.p. Day 7 & 28
G3 IN10018 25mg/kg PO QD
G4 Enhertu+IN10018 3+25mg/kg i.p. Day 7&28 + PO QD
Weight change rate （%）
Day after treatment
-10
0
10
20
30
0
5
10
15
20
25
30
35
40

FIG. 17

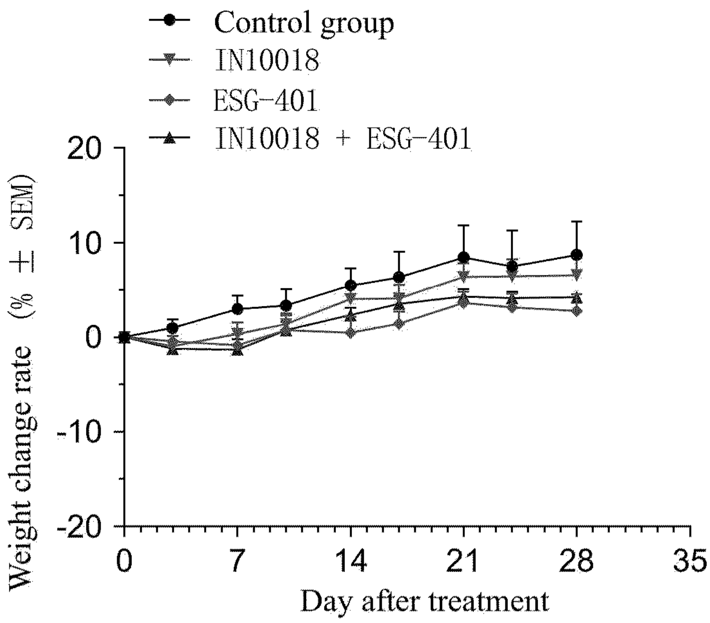
Control group
IN10018
ESG-401
IN10018 + ESG-401
Weight change rate (% ± SEM)
20
10
0
-10
-20
0
7
14
21
28
35
Day after treatment

FIG. 18

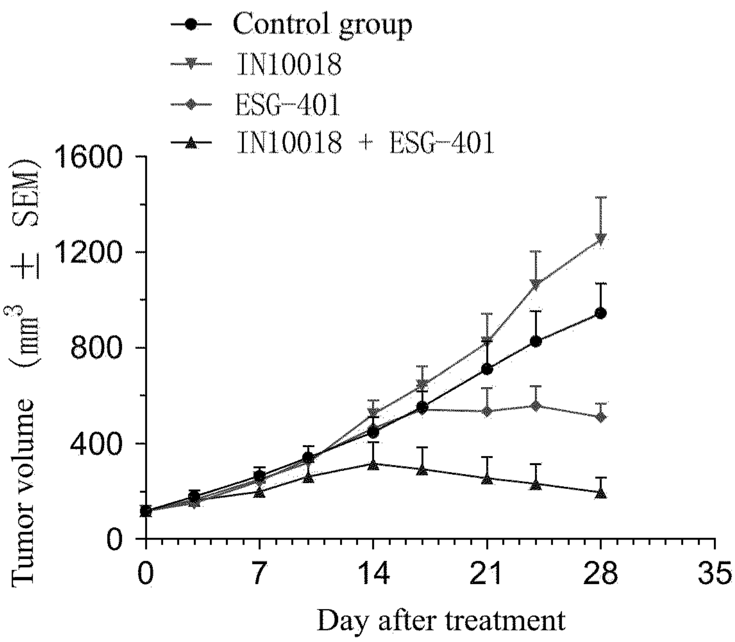
Control group
IN10018
ESG-401
IN10018 + ESG-401
Tumor volume (mm3 ± SEM)
1600
1200
800
400
0
0
7
14
21
28
35
Day after treatment

FIG. 19

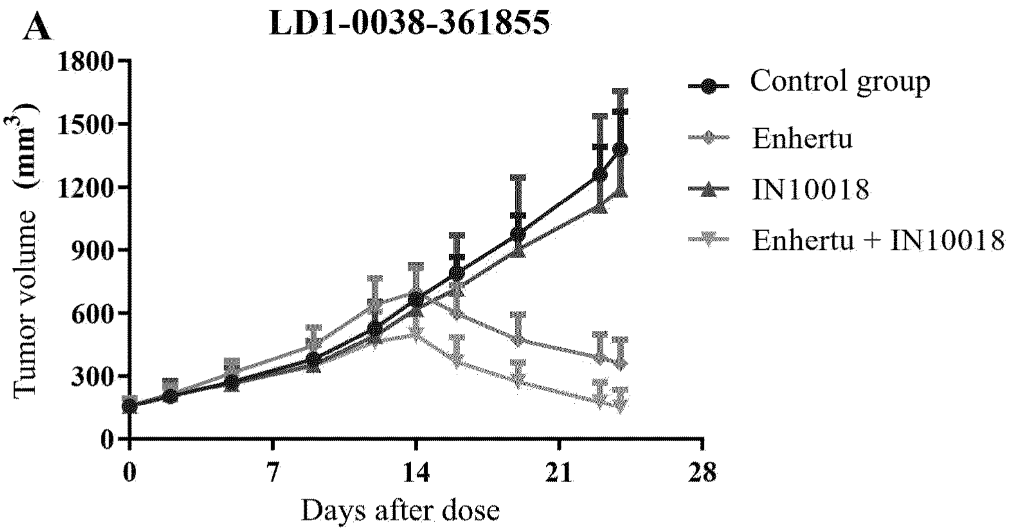
A
LD1-0038-361855
Tumor volume (mm3)
1800
1500
1200
900
600
300
0
0
7
14
21
28
Days after dose
Control group
Enhertu
IN10018
Enhertu + IN10018

FIG. 20

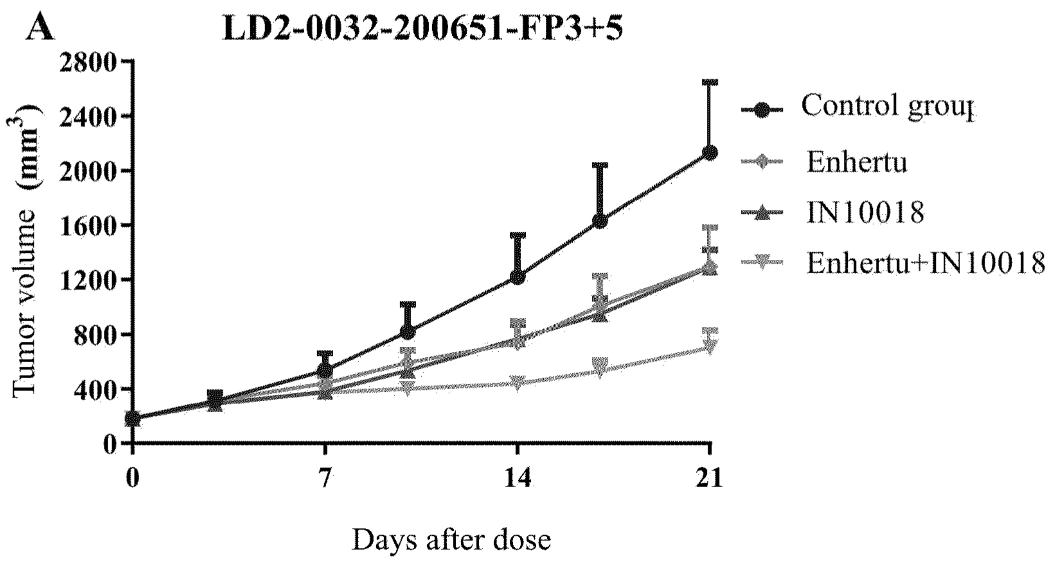
A
LD2-0032-200651-FP3+5
Tumor volume (mm3)
2800
2400
2000
1600
1200
800
400
0
0
7
14
21
Days after dose
Control group
Enhertu
IN10018
Enhertu+IN10018

FIG. 21

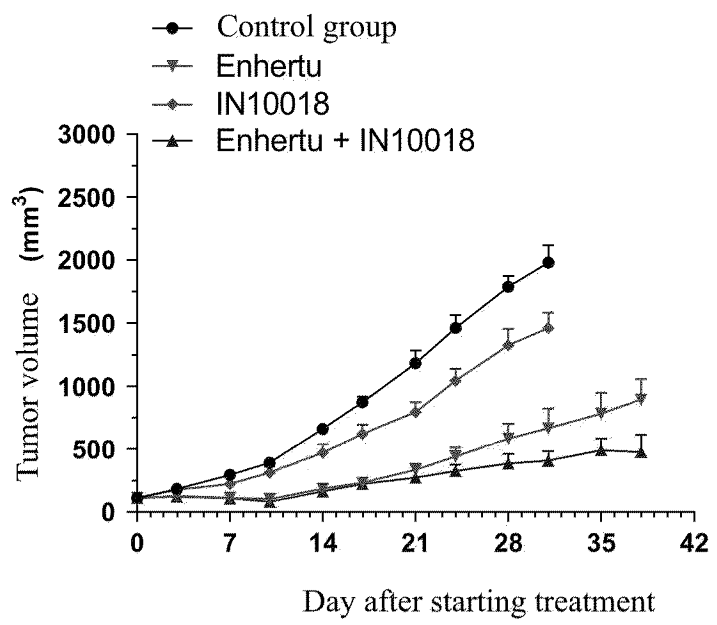
Control group
Enhertu
IN10018
Enhertu + IN10018
Tumor volume (mm3)
3000
2500
2000
1500
1000
500
0
0
7
14
21
28
35
42
Day after starting treatment

FIG. 22

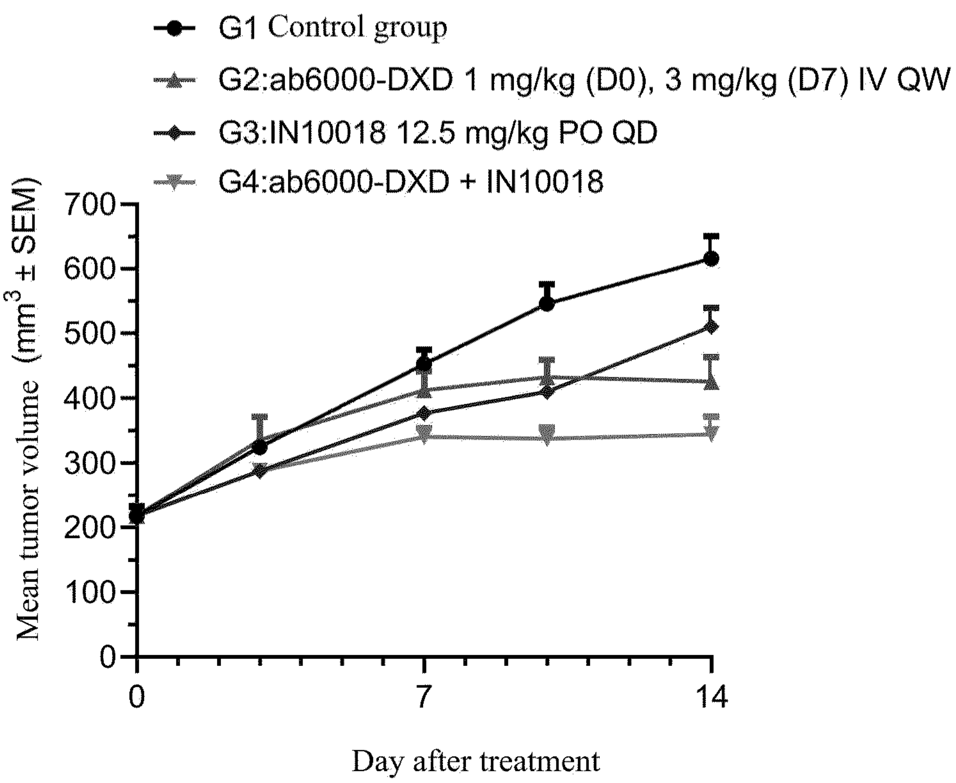
G1 Control group
G2:ab6000-DXD 1 mg/kg (D0), 3 mg/kg (D7) IV QW
G3:IN10018 12.5 mg/kg PO QD
G4:ab6000-DXD + IN10018
Mean tumor volume (mm3 ± SEM)
700
600
500
400
300
200
100
0
0
7
14
Day after treatment

FIG. 23

## INTERNATIONAL SEARCH REPORT

International application No.
**PCT/CN2024/084887**

**A. CLASSIFICATION OF SUBJECT MATTER**

A61K 39/395(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:A61K; A61P

Documentation searched other than minimum documentation to the extent that such documents are included in the fields searched

Electronic data base consulted during the international search (name of data base and, where practicable, search terms used)

CJFD, CNTXT, ENTXTC, ENTXT, VENWFB, WFCCP, WFCD, WFCJ, WFCOA, WFCSTA, WFFCP, WFFD, WFFJ, WFFOA, WFS, WFSTR, PubMed, CNKI, Baidu, ISI Web of Knowledge: IN10018, FAK抑制剂, 抗体, 单抗, 戈沙妥珠单抗, 酒石酸, 酒石酸盐, 浓度, 渗透, 提高, 纤维化, 肿瘤, 肿瘤纤维化, govitecan, SKB264, trop, DS-1062, trop-2, ESG-401

**C. DOCUMENTS CONSIDERED TO BE RELEVANT**

| Category* | Citation of document, with indication, where appropriate, of the relevant passages | Relevant to claim No. |
|---|---|---|
| X | CN 115381956 A (HINOVA PHARMACEUTICALS INC.) 25 November 2022 (2022-11-25) claims 1-4 | 2, 4-13 |
| X | CN 115279376 A (VERASTEM, INC.) 01 November 2022 (2022-11-01) claims 1-2, and description, paragraph 168 | 2, 4-13 |
| X | WU, Y.L. et al. "Focal Adhesion Kinase Inhibitors, A Heavy Punch to Cancer" *Discover Oncology*, Vol. 12, No. (52), 22 November 2021 (2021-11-22), abstract, and section 4, Conclusion | 2, 4-13 |
| A | CN 115429883 A (SHOUYAO HOLDINGS (BEIJING) CO., LTD.) 06 December 2022 (2022-12-06) entire document | 1-13 |
| A | WO 2023036252 A1 (SIGNET THERAPEUTICS INC.) 16 March 2023 (2023-03-16) entire document | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 June 2024** | **19 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/084887**

**C. DOCUMENTS CONSIDERED TO BE RELEVANT**

| Category* | Citation of document, with indication, where appropriate, of the relevant passages | Relevant to claim No. |
|---|---|---|
| A | WU, L.Y. et al. "A Phase Ib Study of IN10018 in Combination with Pegylated Liposomal Doxorubicin (PLD) in Patients with Platinum-Resistant Ovarian Cancer." *2022 ASCO Annual Meeting,* 31 December 2022 (2022-12-31), abstract | 1-13 |

Form PCT/ISA/210 (second sheet) (July 2022)

**INTERNATIONAL SEARCH REPORT**

International application No. **PCT/CN2024/084887**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **3, 4, 6-13**
because they relate to subject matter not required to be searched by this Authority, namely:

Claims 3, 4 and 6-13 relate to a use for increasing the concentration of a macromolecular drug in tumor tissue or a method for treating a tumor, and therefore do not comply with PCT Rule 39.1(iv). A search is performed on the basis of the corresponding pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/084887**

| Patent document cited in search report | Publication date (day/month/year) | Patent family member(s) | Publication date (day/month/year) |
|---|---|---|---|
| CN 115381956 A | 25 November 2022 | AU 2022282251 A1 | 18 January 2024 |
| | | CA 3220357 A1 | 01 December 2022 |
| | | WO 2022247829 A1 | 01 December 2022 |
| | | EP 4349339 A1 | 10 April 2024 |
| | | KR 20240012542 A | 29 January 2024 |
| | | JP 2024519388 A | 10 May 2024 |
| | | BR 112023024596 A2 | 06 February 2024 |
| CN 115279376 A | 01 November 2022 | IL 295117 A | 01 September 2022 |
| | | BR 112022015161 A2 | 11 October 2022 |
| | | AU 2021213753 A1 | 04 August 2022 |
| | | CA 3168648 A1 | 05 August 2021 |
| | | KR 20230011908 A | 25 January 2023 |
| | | WO 2021154929 A1 | 05 August 2021 |
| | | US 2023103007 A1 | 30 March 2023 |
| | | MX 2022009347 A | 15 December 2022 |
| | | EP 4096671 A1 | 07 December 2022 |
| | | EP 4096671 A4 | 28 February 2024 |
| | | JP 2023513015 A | 30 March 2023 |
| CN 115429883 A | 06 December 2022 | None | |
| WO 2023036252 A1 | 16 March 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310343152
- CN 202311276741
- CN 202410283445
- WO 2021225892 A1
- IN 10018
- WO 2010058032 A
- WO 2021225892 A

### Non-patent literature cited in the description

- *CHEMICAL ABSTRACTS*, 1599440-13-7